(19) Europäisches Patentamt
European Patent Office
Office européen des brevets

(11) EP 3 036 307 B1

(12) **EUROPÄISCHE PATENTSCHRIFT**

(45) Veröffentlichungstag und Bekanntmachung des Hinweises auf die Patenterteilung:
**21.06.2017 Patentblatt 2017/25**

(21) Anmeldenummer: **14750445.0**

(22) Anmeldetag: **11.08.2014**

(51) Int Cl.:
***C09K 19/18*** *(2006.01)* ***C09K 19/32*** *(2006.01)*
***C09K 19/34*** *(2006.01)* ***C07C 15/18*** *(2006.01)*
***C07C 15/24*** *(2006.01)* ***C07C 25/24*** *(2006.01)*
***C09K 19/30*** *(2006.01)*

(86) Internationale Anmeldenummer:
**PCT/EP2014/002194**

(87) Internationale Veröffentlichungsnummer:
**WO 2015/024635 (26.02.2015 Gazette 2015/08)**

(54) **ALKINYL-TOLANE, DIESE ENTHALTENDE FLÜSSIGKRISTALLMISCHUNGEN UND KOMPONENTEN FÜR DIE HOCHFREQUENZTECHNIK**

ALKINYL-TOLANES, LIQUID-CRYSTAL MIXTURES CONTAINING THEM AND COMPONENTS FOR HIGH-FREQUENCY TECHNOLOGY

ALCYNYLTOLANES, MÉLANGES DE CRISTAUX LIQUIDES LES CONTENANT ET COMPOSANTS DESTINÉS À LA TECHNOLOGIE HAUTE FRÉQUENCE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.08.2013 EP 13004178**

(43) Veröffentlichungstag der Anmeldung:
**29.06.2016 Patentblatt 2016/26**

(73) Patentinhaber: **Merck Patent GmbH**
**64293 Darmstadt (DE)**

(72) Erfinder:
- **JASPER, Christian**
**65300 Seligenstadt (DE)**
- **BROCKE, Constanze**
**64521 Gross-Gerau (DE)**
- **PAULUTH, Detlef**
**64372 Ober-Ramstadt (DE)**
- **REIFFENRATH, Volker**
**64380 Rossdorf (DE)**
- **MANABE, Atsutaka**
**64625 Bensheim (DE)**

(56) Entgegenhaltungen:
**EP-A2- 0 377 516 DE-A1-102011 112 950**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 036 307 B1

## Beschreibung

Die vorliegende Erfindung betrifft Alkinyl-Tolane, flüssigkristalline Medien enthaltend diese Verbindungen, deren Verwendung für Hochfrequenzbauteile und diese Medien enthaltende Hochfrequenzbauteile, insbesondere Antennen und Phasenschieber, speziell für den Gigahertz- und den Terahertzbereich. Die flüssigkristallinen Medien dienen beispielsweise zur Phasenschiebung von Mikrowellen für abstimmbare 'phased-array' Antennen.

Flüssigkristalline Medien werden seit längerem in elektrooptischen Anzeigen (Liquid Crystal Displays - LCDs) genutzt, um Informationen anzuzeigen.

In neuerer Zeit werden flüssigkristalline Medien jedoch auch vermehrt für die Verwendung in Komponenten, bzw. in Bauteilen, für die Hochfrequenztechnik, insbesondere die Mikrowellentechnik, vorgeschlagen, wie z.B. in DE 10 2004 029 429 A und in JP 2005-120208 (A).

Eine technisch wertvolle Anwendung der flüssigkristallinen Medien in der Hochfrequenztechnik beruht auf ihrer Eigenschaft, dass sie sich durch eine variable Spannung in ihren dielektrischen Eigenschaften steuern lassen, besonders für den Gigahertzbereich. Somit lassen sich abstimmbare Antennen konstruieren, die keine beweglichen Teile beinhalten (A. Gaebler, A. Moessinger, F. Goelden, et al., "Liquid Crystal-Reconfigurable Antenna Concepts for Space Applications at Microwave and Millimeter Waves," International Journal of Antennas and Propagation, vol. 2009, Article ID 876989, 7 Seiten, 2009. doi:10.1155/2009/876989).

Die Druckschrift A. Penirschke, S. Müller, P. Scheele, C. Weil, M. Wittek, C. Hock und R. Jakoby: "Cavity Perturbation Method for Characterization of Liquid Crystals up to 35 GHz", 34th European Microwave Conference - Amsterdam, 545-548 beschreibt unter anderen die Eigenschaften der bekannten, flüssigkristallinen Einzelsubstanz K15 (Merck KGaA, Deutschland) bei einer Frequenz von 9 GHz.

DE 10 2004 029 429 A (vgl. oben) beschreibt die Anwendung von herkömmlichen Flüssigkristallmedien in der Mikrowellentechnik, unter anderem in Phasenschiebern. Dort werden bereits flüssigkristalline Medien bezüglich ihrer Eigenschaften im entsprechenden Frequenzbereich untersucht.

Verbindungen mit einer C-C-Dreifachbindung innerhalb einer Kette von 4 linear angeordneten Benzolringen werden in den Druckschriften JP 05-255151 A und WO 2009/125721 A1 offenbart. Die Verbindungen aus JP 05-255151 A sind teilweise mit Fluorsubstituenten versehen und finden als Komponente von flüssigkristallinen Medien Verwendung. Die in der zweiten Druckschrift offenbarten Verbindungen sind nur an den Molekülenden substituiert und dienen als Bestandteil von Dünnschichttransistoren.

Flüssigkristalline Verbindungen mit sehr hoher optischer Anisotropie und deutlich positiven Werten der dielektrischen Anisotropie sind bisher selten. Verbindungen dieser Art sind bestimmte Bistolane mit einer polaren Endgruppe, wie z.B. in den Druckschriften Shin-Tson Wu et al. Jpn. J. Appl. Phys. 1999, 38, 286-288, Shin-Tson Wu et al. Jpn. J. Appl. Phys. 2000, 39, 38-41, JP 10-45642 A und DE10120024 offenbart.

Die bisher bekannten Zusammensetzungen oder Einzelverbindungen sind jedoch in der Regel mit Nachteilen behaftet. Die meisten von ihnen führen, neben anderen Mängeln, zu unvorteilhaft hohen Verlusten und/oder unzureichenden Phasenverschiebungen bzw. zu geringer Materialgüte. Während beispielsweise manche Einzelverbindungen keine günstigen flüssigkristallinen Phasen und sehr hohe Schmelzpunkte aufweisen, mangelt es wiederum anderen Substanzen an genügend hohen Werten für $\Delta n$ und $\Delta\varepsilon$.

Für die Anwendung in der Hochfrequenztechnik werden flüssigkristalline Medien mit besonderen, bislang eher ungewöhnlichen, ungebräuchlichen Eigenschaften, bzw. Kombinationen von Eigenschaften benötigt.

Somit sind neue Komponenten für flüssigkristalline Medien mit verbesserten Eigenschaften erforderlich. Insbesondere müssen der Verlust im Mikrowellenbereich verringert und die Materialgüte ($\eta$) verbessert werden. Des weiteren finden Anwendungen in der Antennentechnik unter teilweise sich stark ändernden äußeren Rahmenbedingungen statt, wie z.B. starke Temperaturschwankungen. Besonders besteht der Bedarf, das Tieftemperaturverhalten der Bauteile zu verbessern.

Es besteht daher ein erheblicher Bedarf an flüssigkristallinen Medien mit geeigneten Eigenschaften für entsprechende praktische Anwendungen.

Verbindungen der Formel

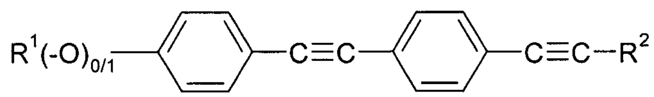

worin $R^1$ und $R^2$ Alkyl bedeuten, werden in EP 0 377 516 (A) offenbart.

Die Verbindung der Formel

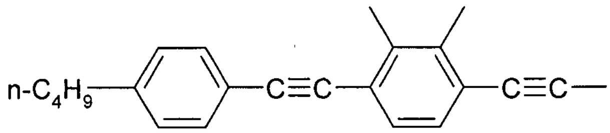

ist aus DE 10 2011 112 950 A1 bekannt.

Überraschend wurde gefunden, dass die erfindungsgemäßen Verbindungen niedrige Schmelzpunkte und hohe Klärpunkte (Übergang der nematischen Phase in die isotrope Phase) aufweisen. Im flüssigkristallinen Bereich sind die Verbindungen überwiegend nematisch oder unterstützen die nematische Phase. Gleichzeitig liegen die optische Anisotropie ($\Delta n$) und die dielektrische Anisotropie ($\Delta\varepsilon$) gleichermaßen bei hohen positiven Werten, wodurch sie sich z.B. für die Anwendung als Hochfrequenzmedium hervorragend eignen. Außerdem weisen die Verbindungen geringe Viskositäten, insbesondere geringe Rotationviskositäten, auf. Dieses ist bei einigen Anwendungen, bei denen es aiüf die Schaltzeiten ankommt, besoners wichtig. Es wurde nun gefunden, dass mit den erfindungsgemäßen Verbindungen flüssigkristalline Medien mit einem breiten nematischen Phasenbereich, gleichzeitig hohen Werten für $\Delta n$ und $\Delta\varepsilon$, und vorteilhaften Hochfrequenzeigenschaften verwirklicht werden können.

Die Erfindung betrifft Verbindungen der Formel I,

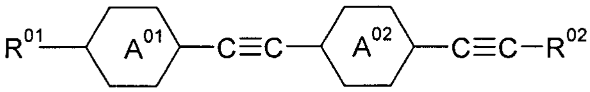 I

worin

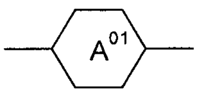

und

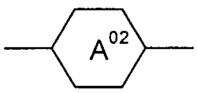

unabhängig voneinander

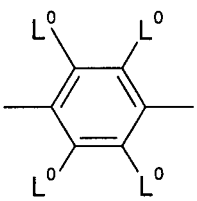 , 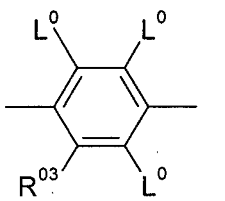 , 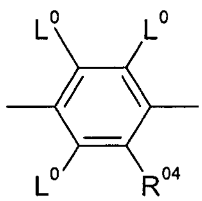 ,

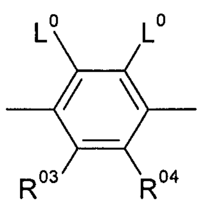 , 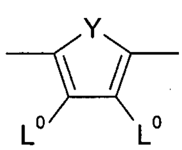 , 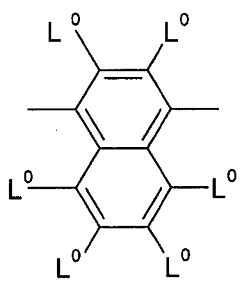 ,

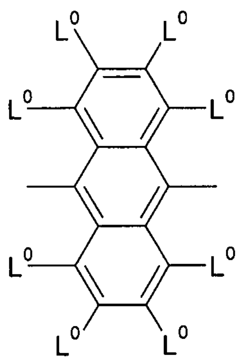,
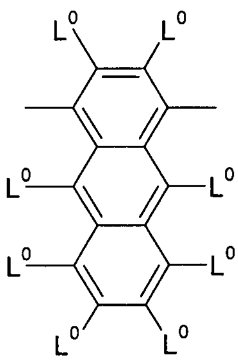,
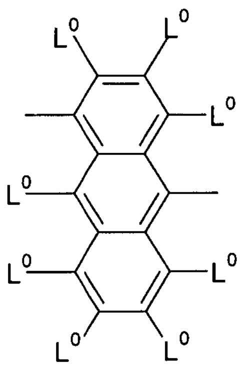

oder

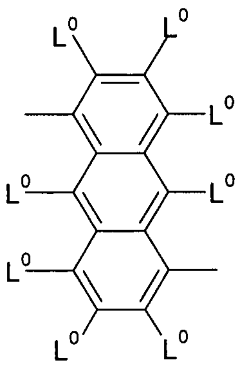,

bevorzugt

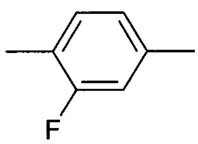,
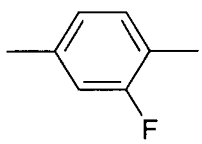,
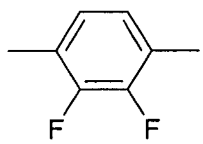,

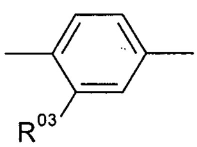,
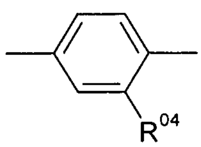
oder
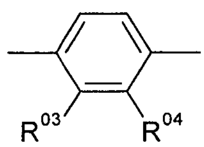,

besonders bevorzugt

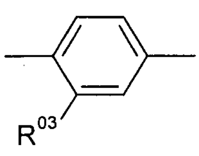,
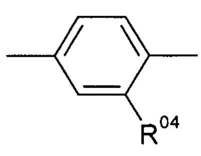
oder
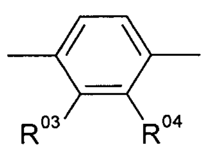,

worin Y S oder O bedeutet,
wobei nur einer von

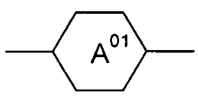

Und

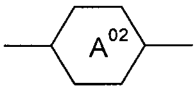

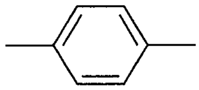

bedeuten kann, und wobei

in den 1,4-Phenylengruppen eine C-H-Gruppe oder mehrere C-H-Gruppen, bevorzugt eine C-H-Gruppe oder zwei C-H-Gruppen, bevorzugt nicht benachbarte, besonders bevorzugt eine C-H-Gruppe, durch N ersetzt sein können und

$L^0$ bei jedem Auftreten unabhängig voneinander H, Br, Cl, F, -CN, -NCS, -SCN, $SF_5$, $C_1$-$C_{10}$ Alkyl, $C_1$-$C_{10}$ Alkoxy, $C_3$-$C_6$ Cycloalkyl oder eine ein- oder mehrfach fluorierte $C_1$-$C_{10}$ Alkyl- oder Alkoxygruppe bedeuten,

$R^{01}$ und $R^{02}$ jeweils unabhängig voneinander einen halogenierten oder unsubstituierten Alkylrest mit 1 bis 15 C-Atomen, wobei in diesen Resten auch eine oder mehrere $CH_2$-Gruppen jeweils unabhängig voneinander durch -C≡C--, -CH=C-, -CF=CF-, -CF=CH-, -CH=CF-, -(CO)O-, -O(CO)-, -(CO)--, -O- oder -S- so ersetzt sein können, dass O- oder S-Atome nicht direkt miteinander verknüpft sind und, gegebenenfalls, unabhängig voneinander $R^{01}$ auch Ethinyl (also -C≡CH) und $R^{02}$ auch H bedeuten können, und

$R^{03}$ und $R^{04}$ jeweils unabhängig voneinander einen halogenierten oder unsubstituierten Alkylrest mit 1 bis 6, bevorzugt mit 1 bis 4, besonders bevorzugt mit 1, 2 oder 3 C-Atomen, wobei in diesen Resten auch eine oder mehrere $CH_2$-Gruppen jeweils unabhängig voneinander durch -C≡C-, -CH=CH-, -CF=CF-, -CF=C-, -CH=CF-, -(CO)O-, -O(CO) -, -(CO)-, -O- oder -S-- so ersetzt sein können, dass O- oder S-Atome nicht direkt miteinander verknüpft sind,

bedeuten,

wobei im Fall, dass

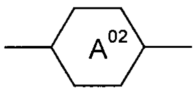

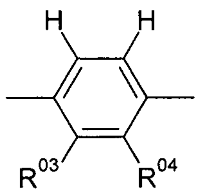

und

$R^{03}$ und $R^{04}$ beide $CH_3$ bedeuten,

$R^{01}$ Alk-1-inyl bedeutet.

Die Doppelbindungen der Teilformeln -CH=CH-, -CH=CF-, -CF=CH- oder -CF=CF- in den entsprechenden Gruppen haben bevorzugt die trans-Konfiguration (E-Konfiguration).

Die erfindungsgemäßen Verbindungen besitzen einen vergleichsweise sehr niedrigen Schmelzpunkt, einen hohen Klärpunkt, eine hohe optische Anisotropie (Δn) und eine deutlich positive dielektrische Anisotropie. Die unerwünschte Rotation der Verbindungen ist eingeschränkt, so dass sie für die Anwendung im Gigahertzbereich besonders geeignet sind. Vorteilhaft ist der relativ geringe Verlustfaktor im Mikrowellenspektrum. Die Verbindungen besitzen allein oder in Mischung mit weiteren mesogenen Komponenten über einen breiten Temperaturbereich eine nematische Phase. Die Gesamtheit dieser Eigenschaften machen sie besonders geeignet für die Verwendung in Bauteilen für die Hochfrequenztechnik, insbesondere in flüssigkristallinen Phasenschiebern. Erfindungsgemäße flüssigkristalline Medien besitzen die entsprechenden Eigenschaften.

Bevorzugte Verbindungen der Formel I sind durch die Auswahl eines oder mehrerer der folgenden Parameter gekennzeichnet:

Besonders bevorzugte Teilstrukturen "-$A^{01}$-≡-$A^{02}$-" sind dabei ausgewählt aus den folgenden Teilstrukturen:

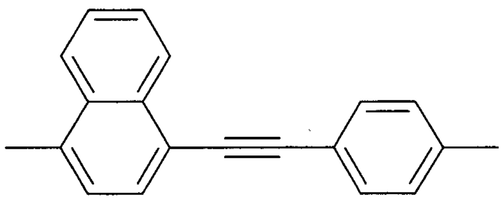,

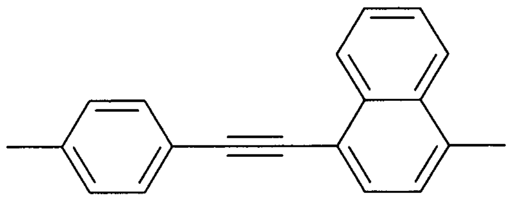,

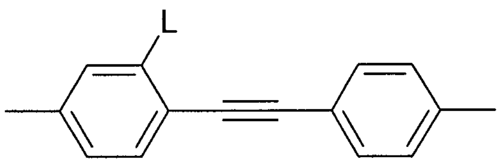,

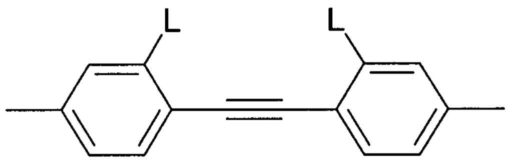,

oder

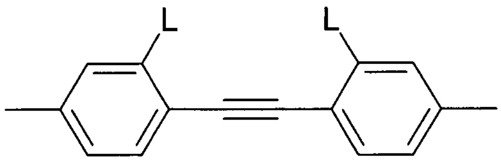

$R^{01}$ bedeutet bevorzugt einen geradkettigen Alkylrest mit 1 bis 15 C-Atomen oder einen Alkinylrest, bevorzugt einen Alk-1-inylrest, mit 2 bis 15 C-Atomen, wobei in diesen Resten auch eine oder mehrere $CH_2$-Gruppen jeweils unabhängig voneinander durch -C≡C-, -CH=CH-, -(CO)O-, -O(CO)-, -(CO)-, -O- so ersetzt sein können, dass O-Atome nicht direkt miteinander verknüpft sind. Bevorzugt ist die Gruppe $R^{01}$ ein Alkyl mit 2 bis 7 C-Atomen.

Die Gruppe L bedeutet bevorzugt Methyl, Ethyl, Propyl, Cyclopropyl oder Cl.

Bevorzugte Ausführungsformen der Erfindung werden daher durch die folgenden beispielhaften Strukturen repräsentiert:

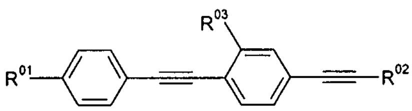

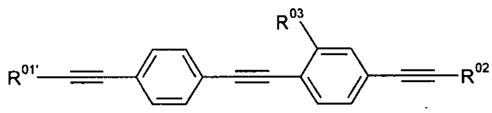

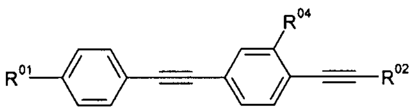

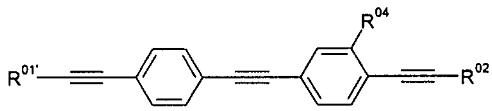

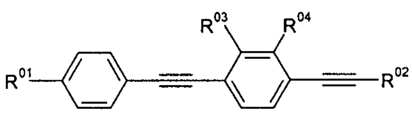

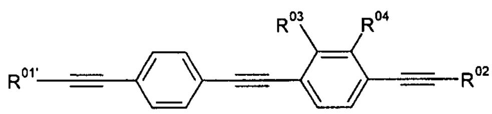

worin $R^{01}$ bis $R^{04}$ wie in Formel I definiert sind, und insbesondere

$R^{01}$ und $R^{01'}$ einen Alkylrest mit 1 bis 7 C-Atomen bei $R^{01}$, und 1 bis 5 C-Atomen, bei $R^{01'}$, beispielsweise einen Methyl- Ethyl-, Propyl-Butyl-, Pentyl- oder Hexylrest,

$R^{02}$ einen Alkylrest mit 1 bis 7 C-Atomen, beispielsweise einen Methyl-Propylrest oder einen Butyl-, Pentyl- oder Hexylrest,

$R^{03}$ einen Alkylrest mit 1 bis 7 C-Atomen, bevorzugt einen Methyl-, Ethyl oder Propylrest und

$R^{04}$ einen Alkylrest mit 1 bis 7 C-Atomen, bevorzugt einen Methyl-, Ethyl oder Propylrest,

bedeutet.

Die Verbindungen der Formel I werden nach an sich bekannten Methoden dargestellt, wie sie in der Literatur (z. B. in den Standardwerken wie Houben-Weyl, Methoden der Organischen Chemie, Georg-Thieme-Verlag, Stuttgart) beschrieben sind und zwar unter Reaktionsbedingungen, die für die genannten Umsetzungen bekannt und geeignet sind. Dabei kann man auch von an sich bekannten, hier nicht näher erwähnten Varianten Gebrauch machen.

Typische Verbindungen der Formel I können vorteilhaft wie an den folgenden beispielhaften Syntheseschemata ersichtlich hergestellt werden (Schemata 1 bis 5):

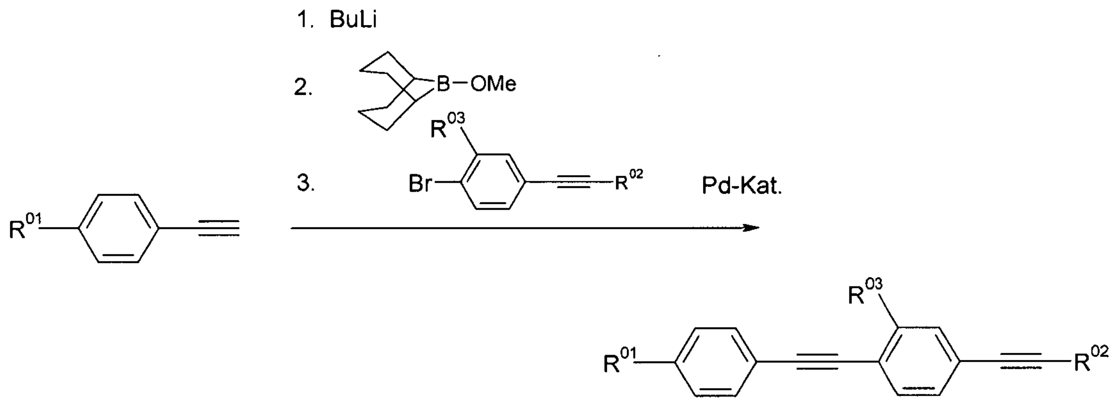


Schema 1: Darstellung von Verbindungen der Formel I, worin, wie in den weiteren Schemata, die Parameter die jeweiligen oben gegeben Bedeutungen haben.

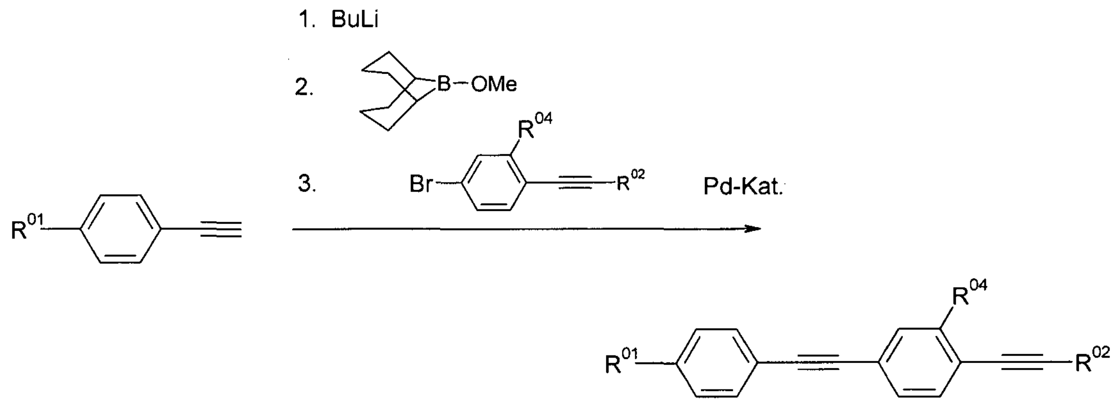


Schema 2: Darstellung von Verbindungen der Formel I.

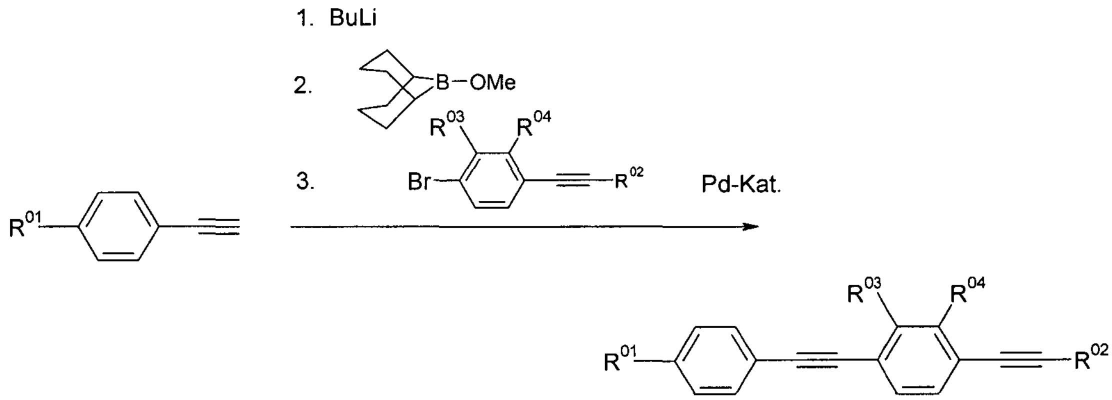


Schema 3: Darstellung von Verbindungen der Formel I.

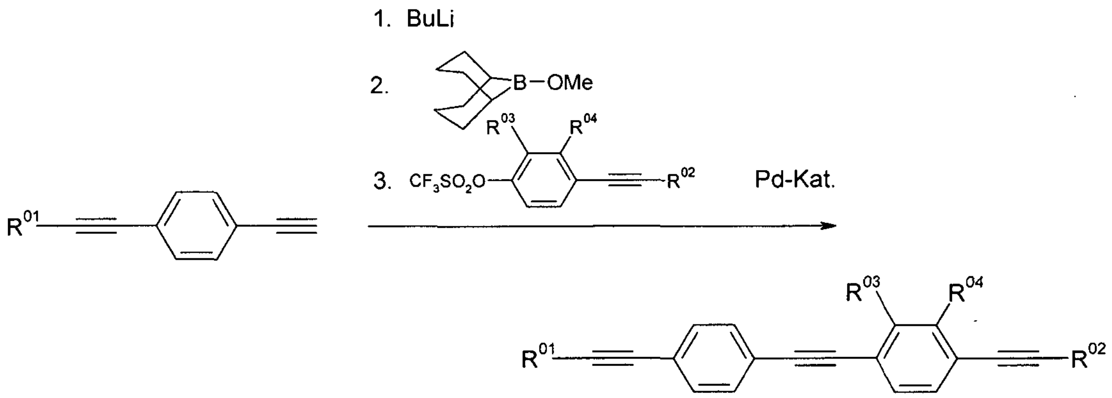


Schema 4: Darstellung von Verbindungen der Formel I.

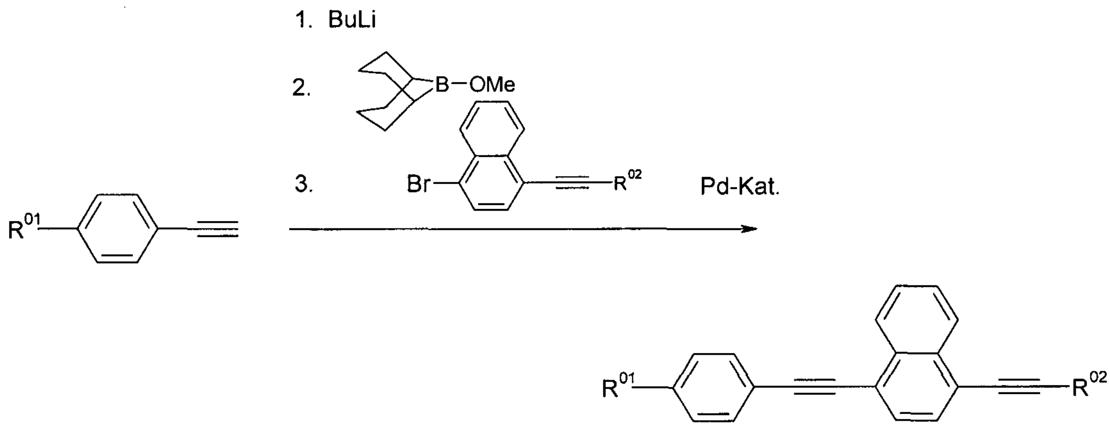


Schema 5: Darstellung von Verbindungen der Formel I worin der Ring A02 1,4-Naphthylen bedeutet.

$R^{01}$ bis $R^{04}$ in Schemata 1 bis 5 haben die Bedeutung, wie für Formel I definiert. In Reaktionsschemata 1 bis 5 wird die Synthese von Verbindungen der Formel I mit bestimmten, bevorzugten Ausführungsformen wiedergegeben. Die Phenylenreste bzw. Naphthylenreste können optional substituiert sein.

Die Parameter sind wie vor- und nachstehend definiert.

Die flüssigkristallinen Medien gemäß der vorliegenden Erfindung enthalten eine oder mehrere Verbindungen der Formel I und optional mindestens eine weitere, vorzugsweise mesogene Verbindung. Das Flüssigkristallmedium enthält daher bevorzugt zwei oder mehr Verbindungen, die vorzugsweise flüssigkristallin sind. Bevorzugte Medien umfassen die bevorzugten Verbindungen der Formel I.

Weitere Komponenten der flüssigkristallinen Medien sind vorzugsweise ausgewählt aus den Verbindungen der Formel II:

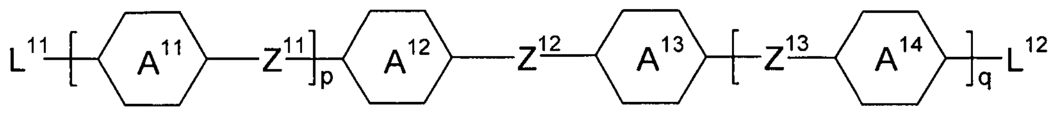 II

worin

| | |
|---|---|
| $L^{11}$ | $R^{11}$ oder $X^{11}$, |
| $L^{12}$ | $R^{12}$ oder $X^{12}$, |
| $R^{11}$ und $R^{12}$ | unabhängig voneinander unfluoriertes Alkyl oder unfluoriertes Alkoxy mit 1 bis 17, bevorzugt mit 3 bis 10, C-Atomen oder unfluoriertes Alkenyl, unfluoriertes Alkinyl, unfluoriertes Alkenyloxy, oder unfluoriertes Alkoxyalkyl mit 2 bis 15, bevorzugt 3 bis 10, C-Atomen, vorzugsweise Alkyl oder unfluoriertes Alkenyl bedeuten, |
| $X^{11}$ und $X^{12}$ | unabhängig voneinander F, Cl, Br, -CN, -NCS, -SCN, -$SF_5$, fluoriertes Alkyl oder fluoriertes Alkoxy mit 1 bis 7 C-Atomen oder fluoriertes Alkenyl, fluoriertes Alkenyloxy oder fluoriertes Alkoxyalkyl mit 2 bis 7 C-Atomen, vorzugsweise fluoriertes Alkoxy, fluoriertes Alkenyloxy, F oder Cl bedeuten, |
| p, q | unabhängig voneinander 0 oder 1, |
| $Z^{11}$ bis $Z^{13}$ | unabhängig voneinander *trans*- -CH=CH-, *trans*--CF=CF-, -C=C- oder eine Einfachbindung bedeuten, |

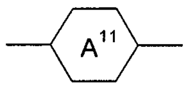 bis 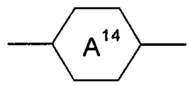

unabhängig voneinander

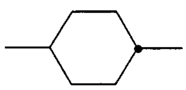, 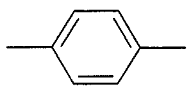, 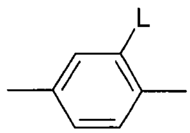,

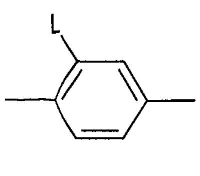, 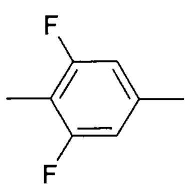, 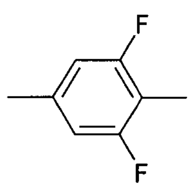,

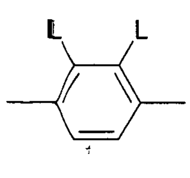, 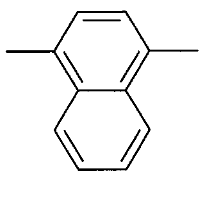,

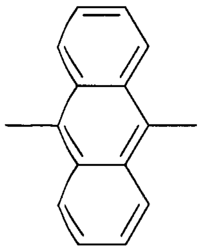 oder 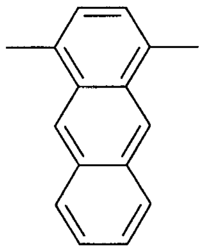 ,

und

L bei jedem Auftreten unabhängig voneinander verzweigtes oder unverzweigtes Alkyl, Alkenyl oder Alkinyl mit 1 bis 12 C-Atomen, worin unabhängig voneinander auch eine oder mehrere "-$CH_2$-"-Gruppen durch O ersetzt sein können, $C_3$-$C_6$ Cycloalkyl, $C_3$-$C_6$ Cycloalkenyl, fluoriertes Alkyl oder Alkenyl, fluoriertes Alkyloxy oder Alkenyloxy, F, Cl, Br, CN, NCS, SCN oder $SF_5$,

bedeuten.

In einer bevorzugten Ausführungsform der vorliegenden Erfindung enthalten die flüssigkristallinen Medien eine oder mehrere Verbindungen der Formel I und eine oder mehrere Verbindungen der Formel II.

Bevorzugt enthalten die flüssigkristallinen Medien gemäß der vorliegenden Anmeldung insgesamt 5 bis 95 %, bevorzugt 10 bis 90 % und besonders bevorzugt 15 bis 80 %, an Verbindungen der Formel I.

Vorzugsweise enthalten die flüssigkristallinen Medien gemäß der vorliegenden Erfindung Verbindungen ausgewählt aus der Gruppe der Verbindungen der Formeln I und II, stärker bevorzugt bestehen sie überwiegend, noch stärker bevorzugt bestehen sie im Wesentlichen und ganz bevorzugt bestehen sie vollständig daraus.

In dieser Anmeldung bedeutet "enthalten" im Zusammenhang mit Zusammensetzungen, dass die betreffende Entität, d.h. das Medium oder die Komponente, die angegebene Komponente oder Komponenten oder Verbindung oder Verbindungen enthält, vorzugsweise in einer Gesamtkonzentration von 10 % oder mehr und ganz bevorzugt von 20 % oder mehr.

"Überwiegend bestehen" bedeutet in diesem Zusammenhang, dass die betreffende Entität 55 % oder mehr, vorzugsweise 60 % oder mehr und ganz bevorzugt 70 % oder mehr der angegebenen Komponente oder Komponenten oder Verbindung oder Verbindungen enthält.

"Im Wesentlichen" bestehen bedeutet in diesem Zusammenhang, dass die betreffende Entität 80 % oder mehr, vorzugsweise 90 % oder mehr und ganz bevorzugt 95 % oder mehr der angegebenen Komponente oder Komponenten oder Verbindung oder Verbindungen enthält.

"Vollständig bestehen" bedeutet in diesem Zusammenhang, dass die betreffende Entität 98 % oder mehr, vorzugsweise 99 % oder mehr und ganz bevorzugt 100,0 % der angegebenen Komponente oder Komponenten oder Verbindung oder Verbindungen enthält.

Bevorzugt enthalten die flüssigkristallinen Medien gemäß der vorliegenden Anmeldung insgesamt 10 bis 100 %, bevorzugt 20 bis 95 % und besonders bevorzugt 25 bis 90 %, an Verbindungen der Formel I und II.

Gemäß der vorliegenden Erfindung werden die Verbindungen der Formel II vorzugsweise in einer Gesamtkonzentration von 10 % bis 90 %, stärker bevorzugt von 15 % bis 85 %, noch stärker bevorzugt von 25 % bis 80 % und ganz bevorzugt von 30 % bis 75 % der Gesamtmischung verwendet.

Die flüssigkristallinen Medien können darüber hinaus weitere Zusätze wie Stabilisatoren, chirale Dotierstoffe und Nanopartikel enthalten. Die einzelnen, zugesetzten Verbindungen werden in Konzentrationen von 0,005 bis 6 %, vorzugsweise von 0,1 bis 3 %, eingesetzt. Die Gesamtkonzentration dieser weiteren Bestandteile liegt im Bereich von 0 % bis 10 %, vorzugsweise 0,1 % bis 6 %, bezogen auf die Gesamtmischung. Dabei werden jedoch die Konzentrationsangaben der übrigen Bestandteile der Flüssigkristallmischungen, also der flüssigkristallinen oder mesogenen Verbindungen, ohne Berücksichtigung der Konzentration dieser Zusatzstoffe angegeben.

Vorzugsweise enthalten die flüssigkristallinen Medien 0 bis 10 Gew.%, insbesondere 0,01 bis 5 Gew.% und besonders bevorzugt 0,1 bis 3 Gew.% an Stabilisatoren. Vorzugsweise enthalten die Medien einen oder mehrere Stabilisatoren ausgewählt aus 2,6-Di-tert-butylphenolen, 2,2,6,6-Tetramethylpiperidinen oder 2-Benzotriazol-2-yl-phenolen. Diese Hilfsstoffe sind dem Fachmann bekannt und kommerziell erhältlich, z. B. als Lichtschutzmittel.

Eine Ausführungsform der Erfindung ist daher auch ein Verfahren zur Herstellung eines Flüssigkristallmediums, das dadurch gekennzeichnet ist, dass eine oder mehrere Verbindungen der Formel I mit einer oder mehreren weiteren Verbindungen und optional mit einem oder mehreren Additiven gemischt wird. Die weiteren Verbindungen sind vorzugsweise ausgewählt aus den Verbindungen der Formel II, wie oben angegeben, und optional einer oder mehreren weiteren Verbindungen.

In der vorliegenden Anmeldung beschreibt der Ausdruck dielektrisch positiv Verbindungen oder Komponenten

mit $\Delta\varepsilon > 3,0$, dielektrisch neutral mit $-1,5 \leq \Delta\varepsilon \leq 3,0$ und dielektrisch negativ mit $\Delta\varepsilon < -1,5$. Die dielektrische Anisotropie der jeweiligen Verbindung wird aus den Ergebnissen einer Lösung von 10 % der jeweiligen einzelnen Verbindung in einer nematischen Host-Mischung bestimmt. Wenn die Löslichkeit der jeweiligen Verbindung in der Host-Mischung weniger als 10 % beträgt, wird die Konzentration auf 5 % reduziert. Die Kapazitäten der Testmischungen werden sowohl in einer Zelle mit homöotroper als auch mit homogener Orientierung bestimmt. Die Schichtdicke beträgt bei beiden Zelltypen ca. 20 μm. Die angelegte Spannung ist eine Rechteckwelle mit einer Frequenz von 1 kHz und einem Effektivwert von typischerweise 0,5 V bis 1,0 V, wird jedoch stets so ausgewählt, dass sie unterhalb der kapazitiven Schwelle für die jeweilige Testmischung liegt.

$\Delta\varepsilon$ ist als $(\varepsilon_{||} - \varepsilon_{\perp})$ definiert, während $\varepsilon_{Durchschnitt}$ $(\varepsilon_{||} + 2\ \varepsilon_{\perp})$ / 3 ist.

Als Host-Mischung wird für dielektrisch positive Verbindungen die Mischung ZLI-4792 und für dielektrisch neutrale sowie für dielektrisch negative Verbindungen die Mischung ZLI-3086 verwendet, beide von Merck KGaA, Deutschland. Die absoluten Werte der dielektrischen Konstanten der Verbindungen werden aus der Änderung der jeweiligen Werte der Host-Mischung bei Zugabe der interessierenden Verbindungen bestimmt. Die Werte werden auf eine Konzentration der interessierenden Verbindungen von 100 % extrapoliert.

Komponenten, die bei der Messtemperatur von 20°C eine nematische Phase aufweisen, werden als solche gemessen, alle anderen werden wie Verbindungen behandelt.

Der Ausdruck Schwellenspannung bezeichnet in der vorliegenden Anmeldung die optische Schwelle und ist für 10 % relativen Kontrast ($V_{10}$) angegeben, der Ausdruck Sättigungsspannung bezeichnet die optische Sättigung und ist für 90 % relativen Kontrast ($V_{90}$) angegeben, in beiden Fällen, soweit nicht ausdrücklich etwas anderes angegeben ist. Die kapazitive Schwellenspannung ($V_0$), auch Freedericks-Schwelle $V_{Fr}$ genannt, wird nur verwendet, wenn dies ausdrücklich genannt ist.

Die in dieser Anmeldung angegebenen Parameterbereiche schließen sämtlich die Grenzwerte ein, wenn nicht ausdrücklich etwas anderes angegeben ist.

Die unterschiedlichen für verschiedene Bereiche von Eigenschaften angegebenen oberen und unteren Grenzwerte ergeben in Kombination miteinander zusätzliche bevorzugte Bereiche.

In der gesamten Anmeldung gelten, wenn nicht ausdrücklich anders angegeben, die folgenden Bedingungen und Definitionen. Alle Konzentrationen sind in Massenprozent angegeben und beziehen sich jeweils auf die Gesamtmischung, alle Temperaturen und alle Temperaturunterschiede sind in Grad Celsius bzw. Differenzgrad angegeben. Alle für Flüssigkristalle typischen physikalischen Eigenschaften werden nach "Merck Liquid Crystals, Physical Properties of Liquid Crystals", Stand Nov. 1997, Merck KGaA, Deutschland, bestimmt und sind für eine Temperatur von 20°C aufgeführt, wenn nicht ausdrücklich anders angegeben. Die optische Anisotropie ($\Delta n$) wird bei einer Wellenlänge von 589,3 nm bestimmt. Die dielektrische Anisotropie ($\Delta\varepsilon$) wird bei einer Frequenz von 1 kHz bestimmt. Die Schwellenspannungen sowie alle anderen elektrooptischen Eigenschaften werden mit bei Merck KGaA, Deutschland, hergestellten Testzellen bestimmt. Die Testzellen für die Bestimmung von $\Delta\varepsilon$ besitzen eine Schichtdicke von circa 20 μm. Bei der Elektrode handelt es sich um eine kreisförmige ITO-Elektrode mit einer Fläche von 1,13 $cm^2$ und einem Schutzring. Die Ausrichtungsschichten sind SE-1211 von Nissan Chemicals, Japan, für homöotrope Ausrichtung ($\varepsilon_{||}$) und Polyimid AL-1054 von Japan Synthetic Rubber, Japan, für homogene Ausrichtung ($\varepsilon_{\perp}$). Die Bestimmung der Kapazitäten erfolgt mit einem Frequenzgang-Analysegerät Solatron 1260 unter Verwendung einer Sinuswelle mit einer Spannung von 0,3 $V_{rms}$. Als Licht wird bei den elektrooptischen Messungen weißes Licht verwendet. Dabei wird ein Aufbau mit einem im Handel erhältlichen Gerät DMS der Fa. Autronic-Melchers, Germany verwendet. Die charakteristischen Spannungen werden unter senkrechter Beobachtung bestimmt. Die Schwellenspannung ($V_{10}$), "Mittgrau-Spannung" ($V_{50}$) und Sättigungsspannung ($V_{90}$) werden für 10 %, 50 % bzw. 90 % relativen Kontrast bestimmt.

Die flüssigkristallinen Medien werden bezüglich ihrer Eigenschaften im Frequenzbereich der Mikrowellen untersucht wie in A. Penirschke et al. "Cavity Perturbation Method for Characterization of Liquid Crystals up to 35GHz", 34th European Microwave Conference - Amsterdam, S. 545-548 beschreiben. Vergleiche hierzu auch A. Gaebler et al. "Direct Simulation of Material Permittivities ...", 12MTC 2009 - International Instrumentation and Measurement Technology Conference, Singapur, 2009 (IEEE), S. 463-467 und DE 10 2004 029 429 A, in der ebenfalls detailliert ein Messverfahren beschrieben wird.

Der Flüssigkristall wird in eine Kapillare aus Polytetrafluorethylen (PTFE) oder Quarzglas gefüllt. Die Kapillare hat einen inneren Radius von 180 μm und einen äußeren Radius von 350 μm. Die effektive Länge beträgt 2,0 cm. Die gefüllte Kapillare wird in die Mitte der zylindrischen Kavität mit einer Resonanzfrequenz von 19 GHz eingebracht. Diese Kavität hat eine Länge von 11,5 mm und einen Radius von 6 mm. Daraufhin wird das Eingangssignal ("source") angelegt und das Ergebnis des Ausgangssignals mit einem kommerziellen Netzwerkanalysator ("vector network analyzer") aufgenommen. Für andere Frequenzen werden die Abmessungen der Kavität entsprechend angepasst.

Aus der Änderung der Resonanzfrequenz und des Q-Faktors, zwischen der Messung mit der mit dem Flüssigkristall gefüllten Kapillare und der Messung ohne der mit dem Flüssigkristall gefüllten Kapillare, wird die dielektrische Konstante und der Verlustwinkel bei der entsprechenden Zielfrequenz mittels der Gleichungen 10 und 11 der zuvor genannten Druckschrift A. Penirschke et al., 34th European Microwave Conference - Amsterdam, S. 545-548 bestimmt,

wie dort beschrieben.

Die Werte für die Komponenten der Eigenschaften senkrecht bzw. and parallel zum Direktor des Flüssigkristalls werden durch Orientierung des Flüssigkristalls in einem Magnetfeld erhalten. Dazu wird das Magnetfeld eines Permanentmagneten verwendet. Die Stärke des Magnetfelds beträgt 0,35 Tesla. Die Orientierung des Magneten wird entsprechend eingestellt und dann entsprechend um 90° gedreht.

Die dielektrische Anisotropie im $\mu$-Wellenbereich ist definiert als

$$\Delta\varepsilon_r \equiv (\varepsilon_{r,||} - \varepsilon_{r,\perp}) .$$

Die Modulierbarkeit bzw. Steuerbarkeit ("tunability", $\tau$) ist definiert als

$$\tau \equiv (\Delta\varepsilon_r / \varepsilon_{r,||}) .$$

Die Materialgüte ($\eta$) ist definiert als

$$\eta \equiv (\tau / \tan \delta_{\varepsilon\, r,Max.}) ,$$

mit dem maximalen dielektrischen Verlustfaktor tan $\delta_{\varepsilon\, r,Max}$:

$$\tan \delta_{\varepsilon\, r,Max.} \equiv \mathrm{Max.}\, \{ \tan \delta_{\varepsilon\, r,\perp} ;\ \tan \delta_{\varepsilon\, r,||} \}$$

der aus dem Maximalwert der gemessenen Werte für tan$\delta_\varepsilon$, hervorgeht.

Die Materialgüte ($\eta$) der bevorzugten Flüssigkristallmaterialien beträgt 6 oder mehr, bevorzugt 7 oder mehr, bevorzugt 10 oder mehr, bevorzugt 15 oder mehr, besonders bevorzugt 25 oder mehr und ganz besonders bevorzugt 30 oder mehr.

Die bevorzugten Flüssigkristallmaterialien haben in den entsprechenden Bauteilen Phasenschiebergüten von 15°/dB oder mehr, bevorzugt von 20°/dB oder mehr, bevorzugt von 30°/dB oder mehr, bevorzugt von 40°/dB oder mehr, bevorzugt von 50°/dB oder mehr, besonders bevorzugt von 80°/dB oder mehr und ganz besonders bevorzugt von 100°/dB oder mehr.

Die erfindungsgemäßen Flüssigkristallmedien weisen bevorzugt nematische Phasen von jeweils mindestens von -20°C bis 80°C, bevorzugt von -30°C bis 85°C und ganz besonders bevorzugt von -40°C bis 100°C auf. Insbesondere bevorzugt reicht die Phase bis 120°C oder mehr, bevorzugt bis 140°C oder mehr und ganz besonders bevorzugt bis 180°C oder mehr. Hierbei bedeutet der Begriff eine nematische Phase aufweisen einerseits, dass bei tiefen Temperaturen bei der entsprechenden Temperatur keine smektische Phase und keine Kristallisation beobachtet wird und andererseits, dass beim Aufheizen aus der nematischen Phase noch keine Klärung auftritt. Die Untersuchung bei tiefen Temperaturen wird in einem Fließviskosimeter bei der entsprechenden Temperatur durchgeführt sowie durch Lagerung in Testzellen, mit einer Schichtdicke von 5 $\mu$m, für mindestens 100 Stunden überprüft. Bei hohen Temperaturen wird der Klärpunkt nach üblichen Methoden in Kapillaren gemessen.

Die Flüssigkristallmedien gemäß der vorliegenden Erfindung weisen bevorzugt einen Klärpunkt von 90°C oder mehr, stärker bevorzugt von 100°C oder mehr, noch stärker bevorzugt von 120°C oder mehr, besonders bevorzugt von 150°C oder mehr und ganz besonders bevorzugt von 170°C oder mehr, auf.

Das $\Delta\varepsilon$ des Flüssigkristallmediums gemäß der Erfindung bei 1 kHz und 20°C beträgt vorzugsweise 1 oder mehr, stärker bevorzugt 2 oder mehr und ganz bevorzugt 3 oder mehr.

Das $\Delta n$ der Flüssigkristallmedien gemäß der vorliegenden Erfindung liegt bei 589 nm ($Na^D$) und 20°C vorzugsweise im Bereich von 0,20 oder mehr bis 0,90 oder weniger, stärker bevorzugt im Bereich von 0,25 oder mehr bis 0,90 oder weniger, noch stärker bevorzugt im Bereich von 0,30 oder mehr bis 0,85 oder weniger und ganz besonders bevorzugt im Bereich von 0,35 oder mehr bis 0,80 oder weniger.

In einer bevorzugten Ausführungsform der vorliegenden Anmeldung beträgt das $\Delta n$ der Flüssigkristallmedien gemäß der vorliegenden Erfindung vorzugsweise 0,50 oder mehr, stärker bevorzugt 0,55 oder mehr.

Ferner sind die erfindungsgemäßen Flüssigkristallmedien durch hohe Anisotropien im Mikrowellenbereich gekennzeichnet. Die Doppelbrechung beträgt z.B. bei ca. 8,3 GHz bevorzugt 0,14 oder mehr, besonders bevorzugt 0,15 oder mehr, besonders bevorzugt 0,20 oder mehr, besonders bevorzugt 0,25 oder mehr und ganz besonders bevorzugt 0,30 oder mehr. Außerdem beträgt die Doppelbrechung bevorzugt 0,80 oder weniger.

Die eingesetzten Flüssigkristalle sind entweder Einzelsubstanzen oder Mischungen. Bevorzugt weisen sie eine nematische Phase auf.

In der vorliegenden Anmeldung, bedeutet, wenn nicht ausdrücklich anders angegeben, der Begriff Verbindungen sowohl eine Verbindung, als auch mehrere Verbindungen.

Bevorzugte Bauelemente, die ein Flüssigkristallmedium oder wenigstens eine Verbindung gemäß der Erfindung enthalten, sind Phasenschieber, Varaktoren, Antennenarrays (z. B. für Funk, Mobilfunk, Radio, Mikrowellen/Radar und sonstige Datenübertragung), 'matching circuit adaptive filters' und andere. Bevorzugt sind Bauteile für die Hochfrequenztechnik, wie oben definiert. Bevorzugt sind außerdem durch unterschiedliche angelegte elektrische Spannungen modulierbare Bauteile. Ganz besonders bevorzugte Bauelemente sind abstimmbare Phasenschieber. In bevorzugten Ausführungsformen werden mehrere Phasenschieber funktionell verbunden, wodurch beispielsweise ein phasengesteuerte Gruppenantenne, in der Regel als 'phased array' Antenne bezeichnet, resultiert. Eine Gruppenantenne nutzt die Phasenverschiebung der in einer Matrix angeordneten Sende- oder Empfangselemente, um durch Interferenz eine Bündelung zu erzielen. Aus einer reihen- oder gitterförmigen parallelen Anordnung von Phasenschiebern lässt sich ein sog. 'phased array' aufbauen, das als abstimmbare oder passive Sende- oder Empfangsantenne für Hochfrequenzen (z. B. Gigahertzbereich) dienen kann. Erfindungsgemäße 'phased array'-Antennen besitzen einen sehr breiten nutzbaren Empfangskegel.

Bevorzugte Anwendungen sind Radarinstallationen und Datenübertragungsgeräte auf bemannten oder unbemannten Fahrzeugen aus dem Bereich Automobil, Schifffahrt, Flugzeuge, Raumfahrt und Satellitentechnik.

Zur Herstellung geeigneter Bauteile für die Hochfrequenztechnik, insbesondere geeigneter Phasenschieber, wird typischerweise ein erfindungsgemäßes flüssigkristallines Medium in rechteckige Kavitäten von weniger als 1 mm Dicke, mehreren Millimetern Breite und mehreren Zentimetern Länge eingebracht. Die Kavitäten besitzen entlang zweier langer Seiten angebrachte, gegenüberliegende Elektroden. Solche Anordnungen sind dem Fachmann vertraut. Durch Anlegen einer variablen Spannung können beim Betreiben der Antenne die dielektrischen Eigenschaften des flüssigkristallinen Mediums abgestimmt werden, um verschiedene Frequenzen oder Richtungen einer Antenne einzustellen.

Der Ausdruck "Halogen" oder "halogeniert" steht für F, Cl, Br und I, besonders für F und Cl und insbesondere für F. Ein halogenierter Alylrest bedeutet daher bevorzugt einen chlorierten oder fluorierten Alkylrest.

Der Ausdruck "Alkyl" umfasst vorzugsweise geradkettige und verzweigte Alkylgruppen mit 1 bis 15 Kohlenstoffatomen, insbesondere die geradkettigen Gruppen Methyl, Ethyl, Propyl, Butyl, Pentyl, Hexyl und Heptyl. Gruppen mit 2 bis 10 Kohlenstoffatomen sind im Allgemeinen bevorzugt.

Der Ausdruck "Alkenyl" umfasst vorzugsweise geradkettige und verzweigte Alkenylgruppen mit 2 bis 15 Kohlenstoffatomen, insbesondere die geradkettigen Gruppen. Besonders bevorzugte Alkenylgruppen sind $C_2$ bis $C_7$-1E-Alkenyl, $C_4$ bis $C_7$-3E-Alkenyl, $C_5$ bis $C_7$-4-Alkenyl, $C_6$ bis $C_7$-5-Alkenyl und $C_7$-6-Alkenyl, insbesondere $C_2$ bis $C_7$-1E-Alkenyl, $C_4$ bis $C_7$-3E-Alkenyl und $C_5$ bis $C_7$-4-Alkenyl. Beispiele weiterer bevorzugter Alkenylgruppen sind Vinyl, 1 E-Propenyl, 1 E-Butenyl, 1 E-Pentenyl, 1 E-Hexenyl, 1 E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Alkoxy" umfasst vorzugsweise geradkettige Reste der Formel $C_nH_{2n+1}$-O-, worin n 1 bis 10 bedeuten. Vorzugsweise ist n 1 bis 6. Bevorzugte Alkoxygruppen sind beispielsweise Methoxy, Ethoxy, n-Propoxy, n-Butoxy, n-Pentoxy, n-Hexoxy, n-Heptoxy, n-Octoxy, n-Nonoxy, n-Decoxy.

Der Ausdruck "Oxaalkyl" bzw. "Alkoxyalkyl" umfasst vorzugsweise geradkettige Reste der Formel $C_nH_{2n+1}$-O-$(CH_2)_m$, worin n und m jeweils unabhängig voneinander 1 bis 10 bedeuten. Vorzugsweise ist n 1 und m 1 bis 6.

Der Ausdruck "fluorierter Alkylrest" umfasst vorzugsweise ein- oder mehrfach fluorierte Reste. Perfluorierte Reste sind eingeschlossen.

Bevorzugt sind $CF_3$, $CH_2CF_3$, $CH_2CHF_2$, $CHF_2$, $CH_2F$, $CHFCF_3$ und $CF_2CHFCF_3$, besonders bevorzugt $CF_3$.

Der Ausdruck "fluorierter Alkoxyrest" umfasst ein- oder mehrfach fluorierte Reste. Perfluorierte Reste sind bevorzugt. Besonders bevorzugt ist der Rest $OCF_3$.

Der Ausdruck "Alk(en/in)ylgruppen, worin ein oder mehrere "-$CH_2$-"-Gruppen durch -O- ersetzt sein können" bezieht sich vorzugsweise auf solche Gruppen, worin eine nicht-terminale $CH_2$-Gruppe ersetzt wird. OH-Gruppen sind in der allgemeinen Bedeutung mit umfasst.

Der Ausdruck "substituiertes Cycloalkyl" umfasst ein- oder mehrfach durch Alkyl substituiertes Cycloalkyl, insbesondere Alkyl mit 1 bis 8 Kohlenstoffatomen.

Der Ausdruck "substituiertes Phenyl" umfasst ein- oder mehrfach durch eine Gruppe wie $R^1$ definiert substituiertes Phenyl, insbesondere durch F, Cl, Alkyl oder Alkoxy substituiertes Phenyl.

In der vorliegenden Anmeldung bedeutet Hochfrequenztechnik Anwendungen mit Frequenzen im Bereich von 1 MHz bis 10 THz, bevorzugt von 1 GHz bis 3 THz, stärker bevorzugt 2 GHz bis 1 THz, insbesondere bevorzugt von 5 bis 300 GHz. Die Anwendung liegt bevorzugt im Mikrowellenspektrum oder angrenzenden, für die Nachrichtenübertragung geeigneten Bereichen, in denen 'phased array'-Module in Sende- und Empfangsantennen zum Einsatz kommen können.

Die erfindungsgemäßen Flüssigkristallmedien bestehen aus einer oder mehreren Verbindungen, vorzugsweise aus 2 bis 30, stärker bevorzugt aus 3 bis 20 und ganz bevorzugt aus 3 bis 16 Verbindungen. Diese Verbindungen werden auf herkömmliche Weise gemischt. In der Regel wird die gewünschte Menge der in der geringeren Menge verwendeten Verbindung in der in der größeren Menge verwendeten Verbindung gelöst. Liegt die Temperatur über dem Klärpunkt der in der höheren Konzentration verwendeten Verbindung, ist die Vervollständigung des Lösungsvorgangs besonders leicht zu beobachten. Es ist jedoch auch möglich, die Medien auf anderen üblichen Wegen, beispielsweise unter Verwendung von so genannten Vormischungen, bei denen es sich z.B. um homologe oder eutektische Mischungen von Verbindungen handeln kann, oder unter Verwendung von so genannten "Multi-Bottle"-Systemen, deren Bestandteile selbst gebrauchsfertige Mischungen sind, herzustellen.

Alle Temperaturen, wie z.B. der Schmelzpunkt T(K,N) bzw. T(K,S), der Übergang von der smektischen (S) zur nematischen (N) Phase T(S,N) und der Klärpunkt T (N,I) der Flüssigkristalle sind in Grad Celsius angegeben. Alle Temperaturdifferenzen sind in Differenzgraden angegeben.

In der vorliegenden Anmeldung und in den folgenden Beispielen sind die Strukturen der Flüssigkristallverbindungen durch Akronyme angegeben, wobei die Transformation in chemische Formeln gemäß folgender Tabellen A und B erfolgt. Alle Reste $C_nH_{2n+1}$ und $C_mH_{2m+1}$ sind geradkettige Alkylreste mit n bzw. m C-Atomen; n, m und k sind ganze Zahlen und bedeuten vorzugsweise 0, 1, 2, 3, 4, 5, 6, 7, 8, 9, 10, 11 oder 12. Die Codierung gemäß Tabelle B versteht sich von selbst. In Tabelle A ist nur das Akronym für den Grundkörper angegeben. Im Einzelfall folgt getrennt von Akronym für den Grundkörper mit einem Strich ein Code für die Substituenten $R^{1*}$, $R^{2*}$, $L^{1*}$ und $L^{2*}$:

| Code für $R^{1*}$, $R^{2*}$, $L^{1*}$, $L^{2*}$, $L^{3*}$ | $R^{1*}$ | $R^{2*}$ | $L^{1*}$ | $L^{2*}$ |
|---|---|---|---|---|
| nm | $C_nH_{2n+1}$ | $C_mH_{2m+1}$ | H | H |
| nOm | $C_nH_{2n+1}$ | $OC_mH_{2m+1}$ | H | H |
| nO.m | $OC_nH_{2n+1}$ | $C_mH_{2m+1}$ | H | H |
| n | $C_nH_{2n+1}$ | CN | H | H |
| nN.F | $C_nH_{2n+1}$ | CN | F | H |
| nN.F.F | $C_nH_{2n+1}$ | CN | F | F |
| nF | $C_nH_{2n+1}$ | F | H | H |
| nCl | $C_nH_{2n+1}$ | Cl | H | H |
| nOF | $OC_nH_{2n+1}$ | F | H | H |
| nF.F | $C_nH_{2n+1}$ | F | F | H |
| nF.F.F | $C_nH_{2n+1}$ | F | F | F |
| $nOCF_3$ | $C_nH_{2n+1}$ | $OCF_3$ | H | H |
| $nOCF_3$.F | $C_nH_{2n+1}$ | $OCF_3$ | F | H |
| n-Vm | $C_nH_{2n+1}$ | $-CH=CH-C_mH_{2m+1}$ | H | H |
| nV-Vm | $C_nH_{2n+1}-CH=CH-$ | $-CH=CH-C_mH_{2m+1}$ | H | H |

Geeignete Mischungskomponenten finden sich in den Tabellen A und B.

**Tabelle A**

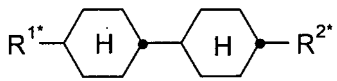

**CCH**

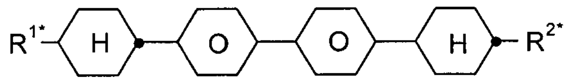

**CBC**

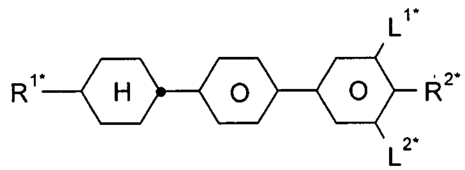

**BCH**

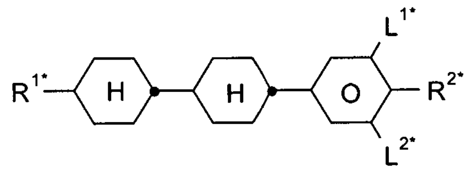

**CCP**

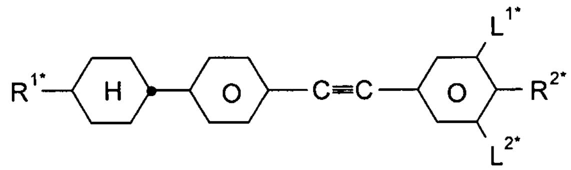

**CPTP**

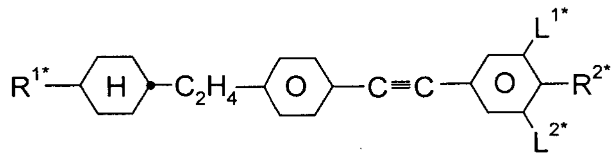

**CEPTP**

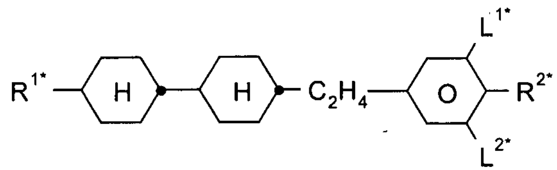

**ECCP**

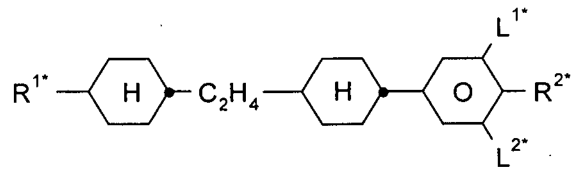

**CECP**

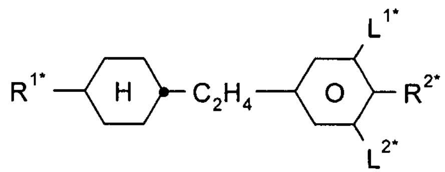

**EPCH**

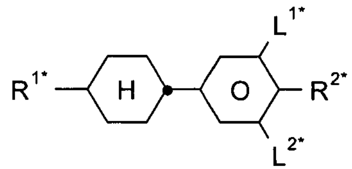

**PCH**

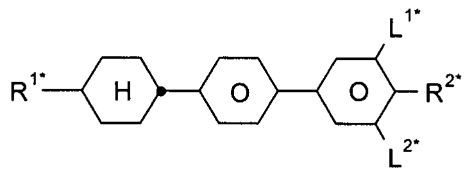

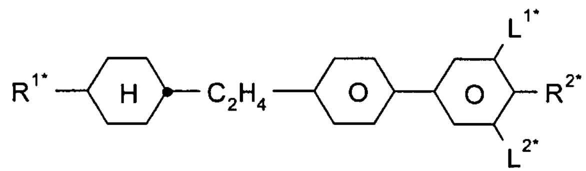

(fortgesetzt)

**PTP**

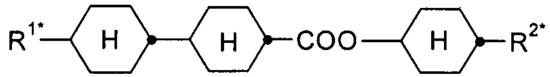

**BECH**

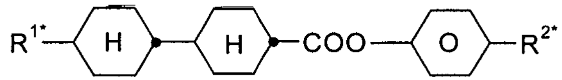

**CH**

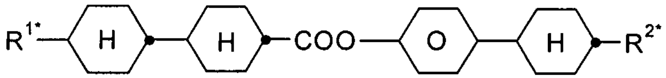

**CP**

**CCPC**

**Tabelle B**

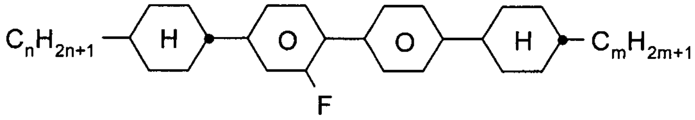

**CBC-nmF**

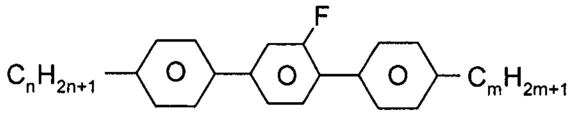

**PGP-n-m**

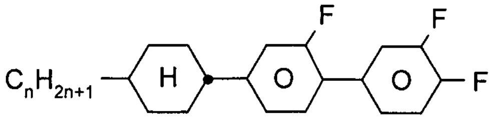

**CGG-n-F**

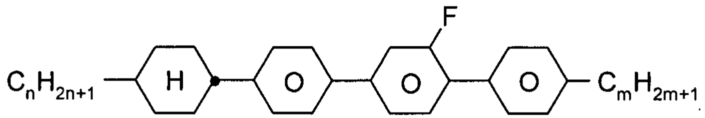

**CPGP-n-m**

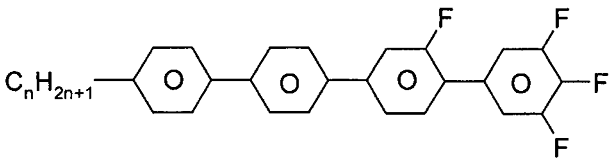

**PPGU-n-F**

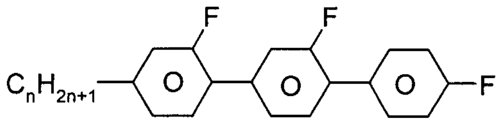

**GGP-n-F**

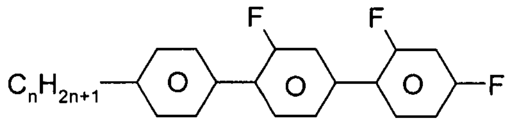

**PGIGI-n-F**

Die folgenden Beispiele erläutern die vorliegende Erfindung, ohne sie in irgendeiner Weise zu beschränken.

Aus den physikalischen Eigenschaften wird dem Fachmann jedoch deutlich, welche Eigenschaften zu erzielen sind und in welchen Bereichen sie modifizierbar sind. Insbesondere ist also die Kombination der verschiedenen Eigenschaften, die vorzugsweise erreicht werden können, für den Fachmann gut definiert.

In der vorliegenden Anmeldung, bedeutet, wenn nicht ausdrücklich anders angegeben, die Pluralform eines Begriffs sowohl die Singularform als auch die Pluralform, und umgekehrt. Weitere Kombinationen der Ausführungsformen und Varianten der Erfindung gemäß der Beschreibung ergeben sich auch aus den angefügten Ansprüchen.

Verwendete Abkürzungen:

| | |
|---|---|
| BuLi | *n*-Butyl-Lithium |
| THF | Tetrahydrofuran |
| MTB | Methyl-tert-butylether |
| $SiO_2$ | Kieselgel |
| RT | Raumtemperatur (ca. 20 °C) |
| Methoxy-9-BBN | B-Methoxy-9-borabicyclo[3.3.1]nonan |

## Beispiele

Die eingesetzten Acetylene und Boronsäuren sind kommerziell erhältlich oder können in Analogie zu bekannten Synthesen hergestellt werden, die dem Fachmann bekannt sind. Die Reste "$C_4H_9$" stehen für unverzweigte n-Butylreste. Entsprechendes gilt für $C_3H_7$, $C_6H_{13}$, etc..

**1. <u>Synthesebeispiel 1:</u>** <u>Synthese von 1-(4-*n*-Butyl-phenylethinyl)-2-methyl-4-propin-1-yl-benzol (entspricht 4-*n*-Butyl-phenyl-(2-Methyl-4-Propin-1-yl- phenyl)-ethin)</u>

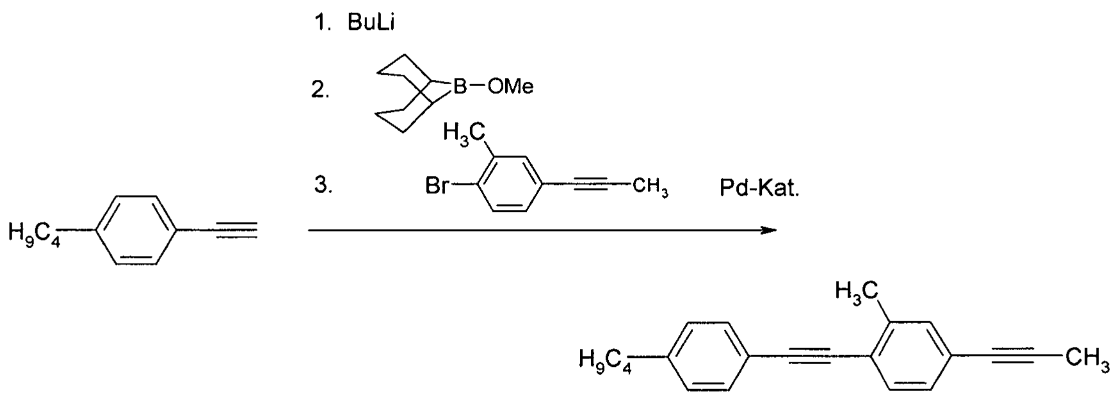


1.1 Stufen 1.1 und 1.2

In einer mit $N_2$ inertisierten 1 l Apparatur werden 6,8 g 1-Butyl-4-ethynyl-benzene in 300 ml THF vorgelegt und auf -78°C gekühlt. Zu dieser Lösung werden 41 ml einer 1 M Lösung von Lithium-bis(trimetylsilyl)amid in *n*-Hexan zugetropft und bei -78°C 30 Min. gerührt. Es entsteht ein klares, gelbliches Reaktionsgemisch. Dann werden 41 ml einer 1 M Lösung von 9-Methoxy-9-BBN zugetropft. Es entsteht ein etwas etwas dunkleres (leicht orangefarbenes), klares Reaktionsgemisch. Dieses wird anschließend 40 Min. bei -78°C gerührt.

1.2 Stufe 1.3

In einer weiteren, mit $N_2$ inertisierten Apparatur werden 6,3 g 1-Bromo-2-methyl-4-prop-1-ynyl-benzene in 400 ml THF vorgelegt und der Katalysator, Bis-(tricyclohexylphosphin)-Palladium-(II)-chlorid, zugegeben. Dann wird bei RT das Reaktionsgemisch der ersten Stufe langsam zugetropft. Anschließend wird unter Rühren, 12 Stunden unter Rückfluß erhitzt.

Anschließend wird wie üblich aufgearbeitet. Im einzelnen wird auf RT abgekühlt, mit Wasser und MTB versetzt und die organische Phase extrahiert, gewaschen und mit Natriumsulfat getrocknet. Man erhält eine rötlich-braune Masse als Rohprodukt. Dieses wird mit Heptan über Kieselgel eluiert. Die so erhaltene, zähe Flüssigkeit wird 2-mal aus der 15-fachen Menge Pentan bei -40°C kristallisiert. Man erhält das Produkt

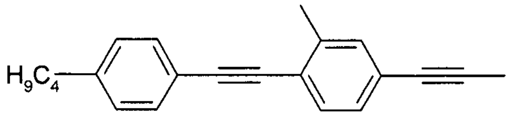

in Form gelblicher Kristalle. Die Reinheit des Produkts wird sowohl mittels GC (99,4 %), als auch mittels HPLC (99,5 %) bestimmt. Das Produkt wird mittels Massenspektrometrie und NMR identifiziert und auf seine physikalischen Eigenschaften untersucht. Diese sind wie folgt. K 39 I; $\Delta\varepsilon$ = +1,6; $\Delta n$ = 0,360 und $\gamma_1$ = 223 mPa·s.

**2. Synthesebeispiel 2:** Synthese von 1-(4-*n*-Butyl-phenylethinyl)-3-methyl-4-propin-1-yl-benzol (entspricht 4-*n*-Butylphenyl-(3-methyl-4-propin-1-yl-phenyl)-ethin)

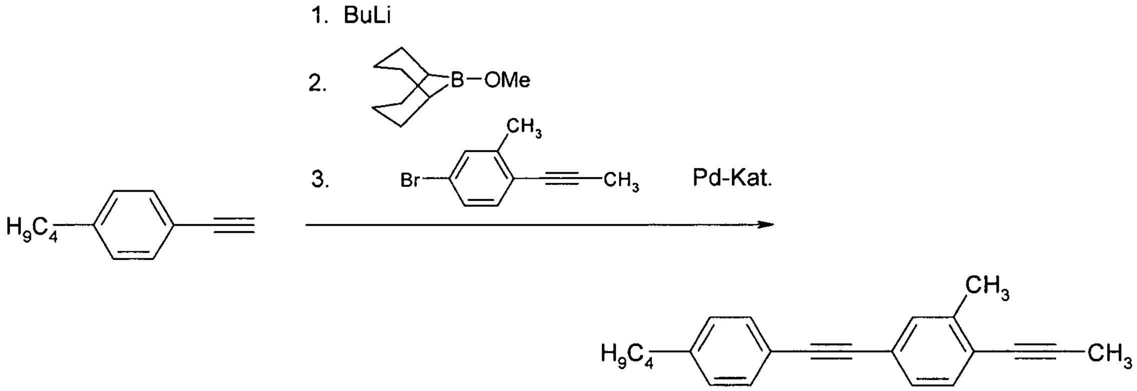


Das Produkt der Stufe 1.2 des Beispiels 1 wird, wie in Stufe 1.3 beschrieben, zu einer Lösung von 1-Bromo-3-methyl-4-prop-1-inyl-benzol gegeben. Man erhält nach Reaktion und Aufarbeitung des Reaktionsgemischs das Produkt

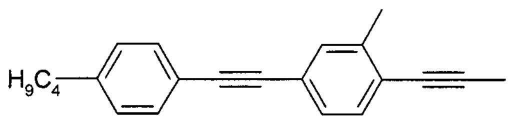

$T_g$ -58 K 24 N (-23,2) I; $\Delta\varepsilon$ = 1,4; $\Delta n$ = 0,347 und $\gamma_1$ = 166 mPa·s.

**3. Vergleichssynthesebeispiel 1:** Synthese von 1-(4-*n*-Butylphenylethinyl)-2,3-di-methyl-4-propin-1-yl-benzol (entspricht 4-*n*-Butylphenyl-(2,3-di-methyl-4-propin-1-yl-phenyl)-ethin)

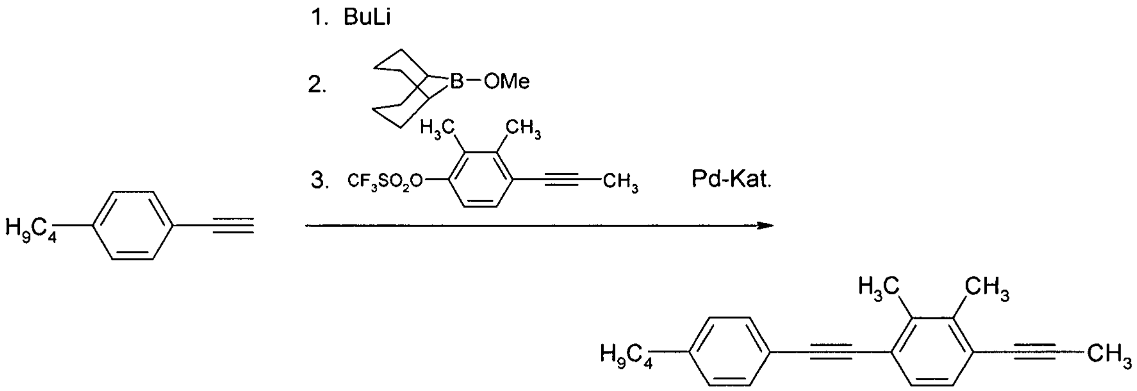


Das Produkt der Stufe 1.2 des Beispiels 1 wird, wie in Stufe 1.3 beschrieben, zu einer Lösung von 1-Bromo-2,3-di-methyl-4-prop-1-inyl-benzol gegeben. Man erhält nach Reaktion und Aufarbeitung des Reaktionsgemischs das Produkt

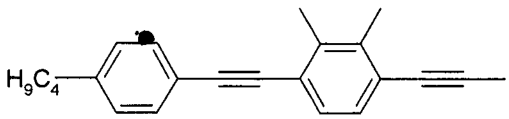

K 72 N (51) I; $\Delta\varepsilon$ = 2,6; $\Delta n$ = 0,350 und $\gamma_1$ = 224 mPa·s.

**4. Synthesebeispiel 3:** Synthese von 1-(4-*n*-But-1in-1-yl-phenylethinyl)-2,3-dimethyl-4-propin-1-yl-benzol (entspricht 4-*n*-But-1-in-1-yl-phenyl-(2,3-dimethyl-4-propin-1-yl-phenyl)-ethin)

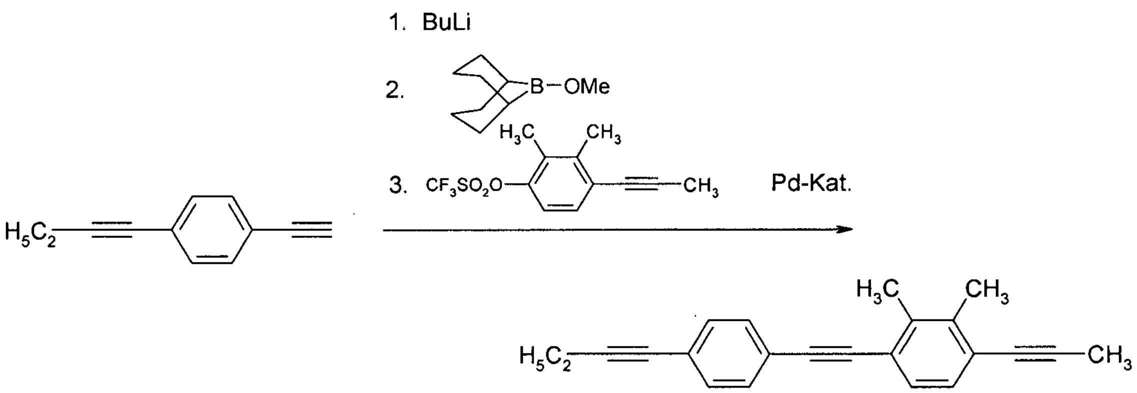


Die Umsetzung erfolgt analog zu der in Beispiel 1 beschriebenen. Man erhält nach Reaktion und Aufarbeitung des Reaktionsgemischs das Produkt

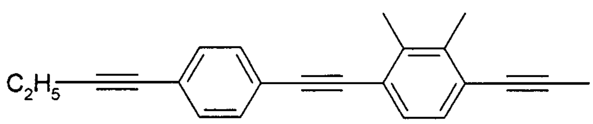

K 119 N 127,7 I; $\Delta\varepsilon$ = 2,3; $\Delta n$ = 0,494 und $\gamma_1$ = 610 mPa·s.

Analog, beziehungsweise vergleichbar werden synthetisiert:

5. **Substanzbeispiel 4:**

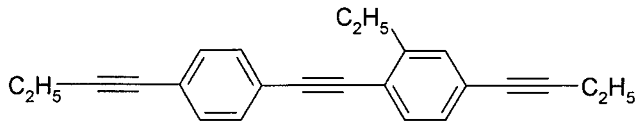

K 22 I; Δε = 0,5; Δn = 0,408 und $\gamma_1$ = 482 mPa·s.

6. **Substanzbeispiel 5:**

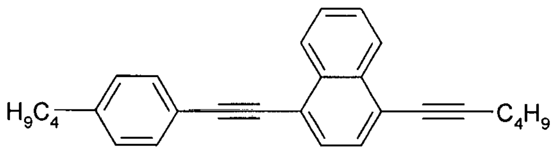

$T_g$ -52 I; Δε = 0,7; Δn = 0,270 und $\gamma_1$ = 356 mPa·s.

7. **Substanzbeispiel 6:**

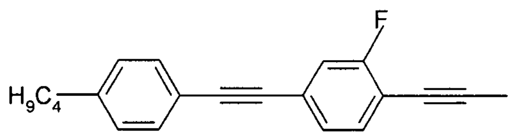

K 87 I; Δε = 1,8; Δn = 0,350 und $\gamma_1$ = 108 mPa·s.

**Mischungsbeispiele**

**Mischungsbeispiel 1**

Es wird ein Flüssigkristallmedium M-1 mit der Zusammensetzung und den Eigenschaften wie in der folgenden Tabelle angegeben hergestellt. Die Verbindung (**1**) (Nr. 15) stammt aus dem Synthesebeispiel 1.

| Zusammensetzung | | | Physikalische Eigenschaften | | |
|---|---|---|---|---|---|
| Verbindung | | | T(N,I) | = 90,2 | °C |
| Nr. | Abkürzung | | | | |
| 1 | BCH-3F.F | 10,8 % | | | |
| 2 | BCH-5F.F | 9,0 % | Δn (20°C, 589,3 nm) | = 0,123 | |
| 3 | ECCP-30CF3 | 4,5 % | | | |
| 4 | ECCP-50CF3 | 4,5 % | Δε (20°C, 1 kHz) | = 4,8 | |
| 5 | CBC-33F | 1,8 % | | | |
| 6 | CBC-53F | 1,8 % | $\gamma_1$ (20°C) | = 139 | mPa·s |
| 7 | CBC-55F | 1,8 % | | | |
| 8 | PCH-6F | 7,2 % | | | |
| 9 | PCH-7F | 5,4 % | | | |
| 10 | CCP-20CF3 | 7,2 % | | | |

(fortgesetzt)

| Zusammensetzung | | | Physikalische Eigenschaften |
|---|---|---|---|
| 11 | CCP-30CF3 | 10,8 % | |
| 12 | CCP-40CF3 | 6,3 % | |
| 13 | CCP-50CF3 | 9,9 % | |
| 14 | PCH-5F | 9,0 % | |
| 15 | (1) | 10,0 % | |
| $\Sigma$ | | 100,0 % | |

Diese Mischung wird für Anwendungen im Mikrowellenbereich, insbesondere für Phasenschieber bzw. für 'phased array' Antennen verwendet.

Zum Vergleich wird ein Medium CM-0 ohne die Komponente (**1**) aus den Verbindungen Nr. 1-14 des Mediums M-1 hergestellt, wobei die Verbindungen Nr. 1-14 in den gleichen relativen Mengen enthalten sind.

**Mischungsbeispiele 2 bis 6 und Vergleichsmischungsbeispiel CM-0**

Es werden Flüssigkristallmedien M-2 bis M-6 mit der Zusammensetzung von M-1 hergestellt, mit dem Unterschied, dass für M-2 anstelle der Verbindung (**1**) die Verbindung (**2**) aus Synthesebeispiel 2 und für M-3 anstelle der Verbindung (**1**) die Verbindung (**3**) aus Synthesebeispiel 3 eingesetzt wird u.s.w..

Die Ergebnisse zu den Mischungsbeispielen finden sich in der nachfolgenden Tabelle.

Tabelle 1: Allgemeine physikalische Eigenschaften der Mischungen

| | Eigenschaft | | | |
|---|---|---|---|---|
| Mischung | $T(N,I)/°C_I$ | $\Delta n$ | $\Delta\varepsilon$ | $\gamma_1$/ mPa·s |
| M-1 | 90,2 | 0,123 | 4,8 | 139 |
| M-2 | 88,8 | 0,122 | 4,8 | 137 |
| M-3 | 100,2 | 0,137 | 5,0 | 153 |
| M-4 | 83,1 | 0,127 | 4,7 | 150 |
| M-5 | 85,3 | 0,114 | 4,8 | 139 |
| M-6 | 90,0 | 0,122 | 5,0 | 129 |
| CM-0 | 92,0 | 0,097 | 5,2 | n.z.b. |
| n.z.b.: noch zu bestimmen | | | | |

Tabelle 2: Eigenschaften der Mischungen bei 19 GHz (20°C)

| | Eigenschaft | | | | | |
|---|---|---|---|---|---|---|
| Mischung | $\varepsilon_{r,\|\|}$ | $\varepsilon_{r,\perp}$ | $\tau$ | tan $\delta_{\varepsilon, r,\|\|}$ | tan $\delta_{\varepsilon, r,\perp}$ | $\eta$ |
| M-1 | 2,63 | 2,29 | 0,131 | 0,0046 | 0,0118 | 11,3 |
| M-2 | 2,56 | 2,26 | 0,118 | 0,0044 | 0,0118 | 10,0 |
| M-3 | 2,68 | 2,32 | 0,135 | 0,0046 | 0,0120 | 11,3 |
| M-4 | n.z.b. | n.z.b. | n.z.b. | n.z.b. | n.z.b. | n.z.b. |
| M-5 | n.z.b. | n.z.b. | n.z.b. | n.z.b. | n.z.b. | n.z.b. |
| M-6 | n.z.b. | n.z.b. | n.z.b. | n.z.b. | n.z.b. | n.z.b. |
| CM-0 | 2,56 | 2,29 | 0,107 | 0,0049 | 0,0126 | 8,50 |
| $\tau$ = Steuerbarkeit; $\eta$ = Materialgüte; tan $\delta_{\varepsilon, r}$ = dielektrische Verlustfaktoren, n.z.b.: noch zu bestimmen | | | | | | |

Die Steuerbarkeit τ und die Materialgüte η sind für die Mischungen M-1 bis M-6 gegenüber der Vergleichsmischung CM-0 deutlich verbessert.

## Patentansprüche

1. Verbindungen der Formel **I**

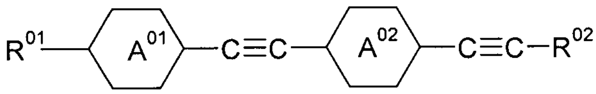

I

worin

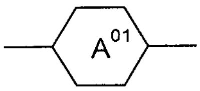

und

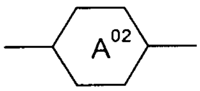

unabhängig voneinander

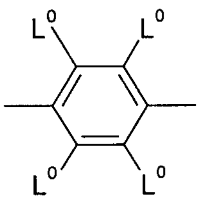
,
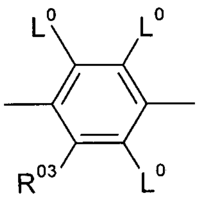
,
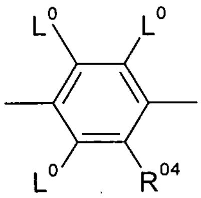
,

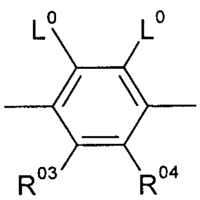
,
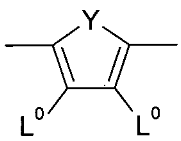
,
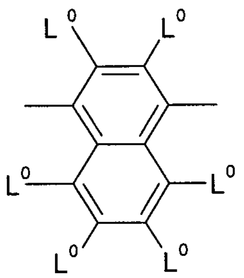
,

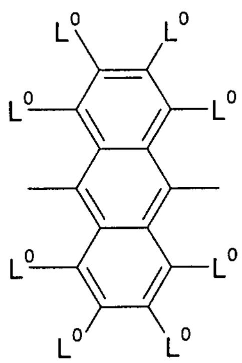,
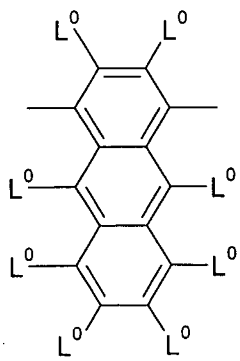,
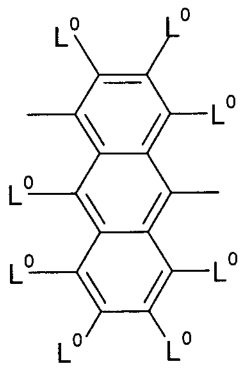

oder

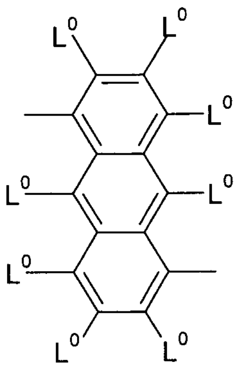,

worin Y S oder O bedeutet, wobei nur einer von

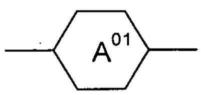

und

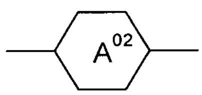
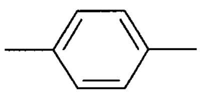

bedeuten kann, und in den 1,4-Phenylengruppen eine C-H-Gruppe oder zwei C-H-Gruppen, durch N ersetzt sein können und

$L^0$ bei jedem Auftreten unabhängig voneinander H, Br, Cl, F, -CN, -NCS, -SCN, $SF_5$, $C_1$-$C_{10}$ Alkyl, $C_1$-$C_{10}$ Alkoxy, $C_3$-$C_6$ Cycloalkyl oder eine ein- oder mehrfach fluorierte $C_1$-$C_{10}$ Alkyl- oder Alkoxygruppe bedeuten,
$R^{01}$ und $R^{02}$ jeweils unabhängig voneinander einen halogenierten oder unsubstituierten Alkylrest mit 1 bis 15 C-Atomen, wobei in diesen Resten auch eine oder mehrere $CH_2$-Gruppen jeweils unabhängig voneinander durch -C≡C-, -CH=CH-, -CF=CF-, -CF=CH-, -CH=CF-, -(CO)O-, -O(CO)-, -(CO)-, -O- oder -S- so ersetzt sein können, dass O- oder S-Atome nicht direkt miteinander verknüpft sind und, gegebenenfalls, unabhängig voneinander $R^{01}$ auch Ethinyl (also -C≡CH)und $R^{02}$ auch H bedeuten können, und
$R^{03}$ und $R^{04}$ jeweils unabhängig voneinander einen halogenierten oder unsubstituierten Alkylrest mit 1 bis 6, bevorzugt mit 1 bis 4, besonders bevorzugt mit 1, 2 oder 3 C-Atomen, wobei in diesen Resten auch eine oder mehrere $CH_2$-Gruppen jeweils unabhängig voneinander durch -C≡C-, -CH=CH-, -CF=CF-, -CF=CH-, -CH=CF-, -(CO)O-, -O(CO)-, -(CO)-, -O- oder -S- so ersetzt sein können, dass O- oder S-Atome nicht direkt miteinander verknüpft sind,

bedeuten,
wobei im Fall, dass

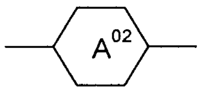

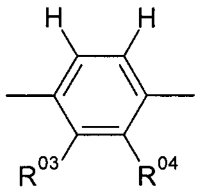

und

$R^{03}$ und $R^{04}$ beide $CH_3$ bedeuten,
$R^{01}$ Alk-1-inyl bedeutet.

2. Verbindungen nach Anspruch 1, **dadurch gekennzeichnet, dass**

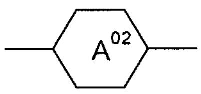

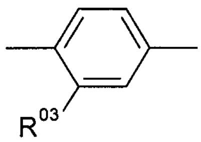

,

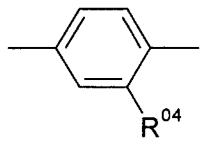

oder

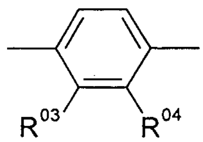

bedeutet.

3. Verbindungen nach Anspruch 1 oder 2, **dadurch gekennzeichnet, dass**

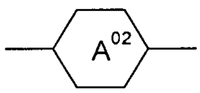

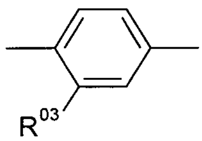

oder

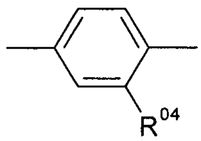

bedeutet.

4. Verbindungen nach einem oder mehreren der Ansprüche 1 bis 3 ausgewählt aus der Gruppe der Verbindungen der folgenden Formeln

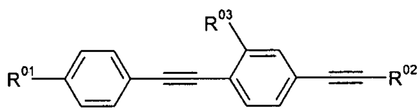

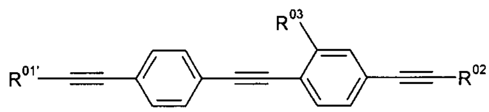

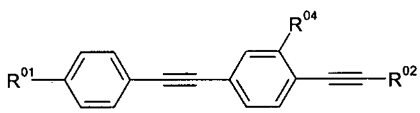

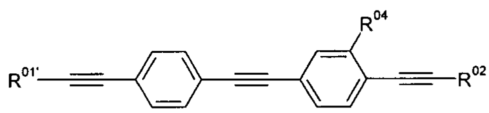

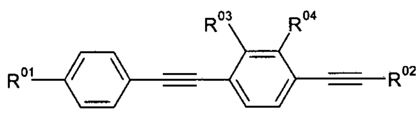

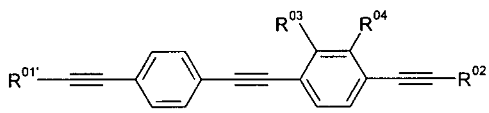

worin $R^{01}$ bis $R^{04}$ die in Anspruch 1 gegebenn Bedeutungen haben.

5. Verbindungen nach einem oder mehreren der Ansprüche 1 bis 4, **dadurch gekennzeichnet, dass** $R^{01}$ Alk-1-inyl

bedeutet.

6. Flüssigkristallmedium, **dadurch gekennzeichnet, dass** es eine oder mehrere Verbindungen der Formel I nach einem der Ansprüche 1 bis 5 enthält.

7. Flüssigkristallmedium nach Anspruch 6, **dadurch gekennzeichnet, dass** es zusätzlich eine oder mehrere Verbindungen ausgewählt aus den Verbindungen der Formel II enthält:

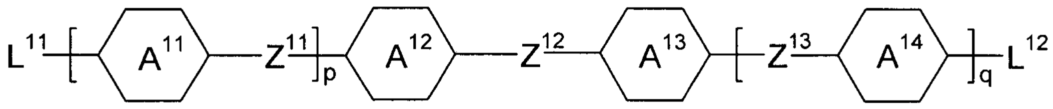 II

worin

$L^{11}$ $R^{11}$ oder $X^{11}$,
$L^{12}$ $R^{12}$ oder $X^{12}$,
$R^{11}$ und $R^{12}$ unabhängig voneinander unfluoriertes Alkyl oder unfluoriertes Alkoxy mit 1 bis 17 C-Atomen oder unfluoriertes Alkenyl, unfluoriertes Alkinyl, unfluoriertes Alkenyloxy, oder unfluoriertes Alkoxyalkyl mit 2 bis 15 C-Atomen,
$X^{11}$ und $X^{12}$ unabhängig voneinander F, Cl, Br, -CN, -NCS, -SCN, -$SF_5$, fluoriertes Alkyl oder fluoriertes Alkoxy mit 1 bis 7 C-Atomen oder fluoriertes Alkenyl, fluoriertes Alkenyloxy oder fluoriertes Alkoxyalkyl mit 2 bis 7 C-Atomen,
p, q unabhängig 0 oder 1,
$Z^{11}$ bis $Z^{13}$ unabhängig voneinander *trans*- -CH=CH-, *trans*--CF=CF-, -C=C- oder eine Einfachbindung, und

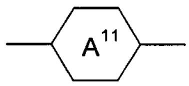 bis 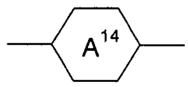

unabhängig voneinander

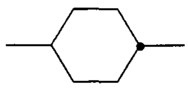 , 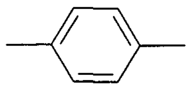 , 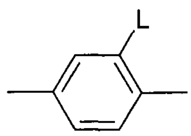 ,

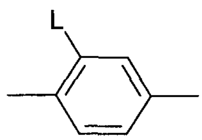 , 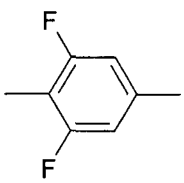 , 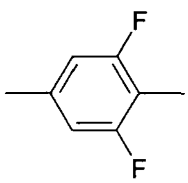 ,

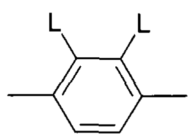 , 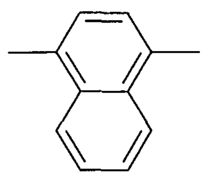 ,

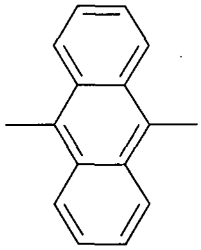 oder 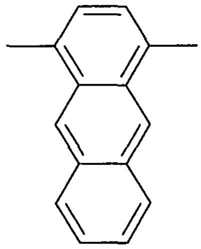 ,

und

L bei jedem Auftreten unabhängig vonainander verzweigtes oder unverzweigtes Alkyl, Alkenyl oder Alkinyl mit 1 bis 12 C-Atomen, worin unabhängig voneinander auch eine oder mehrere "-$CH_2$-"-Gruppen durch O ersetzt sein können, $C_3$-$C_6$ Cycloalkyl, $C_3$-$C_6$ Cycloalkenyl, fluoriertes Alkyl oder Alkenyl, fluoriertes Alkyloxy oder Alkenyloxy, F, Cl, Br, CN, NCS, SCN oder $SF_5$,

bedeutet.

**8.** Flüssigkristallmedium nach Anspruch 6 oder 7, **dadurch gekennzeichnet, dass** die Konzentration der Verbindungen der Formel I im Medium im Bereich von insgesamt 5 % bis 95 % liegt.

**9.** Verwendung der Verbindungen der Formel I nach einem der Ansprüche 1 bis 5 in einem flüssigkristallinen Medium.

**10.** Verwendung der Verbindungen der Formel I nach einem der Ansprüche 1 bis 5 oder eines flüssigkristallinen Mediums nach einem der Ansprüche 6 bis 8 in einem Bauteil für die Hochfrequenztechnik.

**11.** Verfahren zur Herstellung eines Flüssigkristallmediums nach einem oder mehreren der Ansprüche 6 bis 8, **dadurch gekennzeichnet, dass** eine oder mehrere Verbindungen der Formel I mit einer oder mehreren weiteren Verbindungen und optional mit einem oder mehreren Additiven gemischt wird.

**12.** Bauteil für die Hochfrequenztechnik, **dadurch gekennzeichnet, dass** es ein Flüssigkristallmedium nach einem oder mehreren der Ansprüche 6 bis 8 enthält.

**13.** Bauteil nach Anspruch 12, **dadurch gekennzeichnet, dass** es sich um einen Phasenschieber oder um mehrere funktionell verbundene Phasenschieber handelt.

**14.** 'Phased array' Antenne, **dadurch gekennzeichnet, dass** sie ein oder mehrere Bauteile nach Anspruch 12 oder 13 enthält.

**15.** Verwendung eines Flüssigkristallmediums nach einem oder mehreren der Ansprüche 6 bis 8 in einem Bauteil für die Hochfrequenztechnik.

## Claims

**1.** Compounds of the formula I

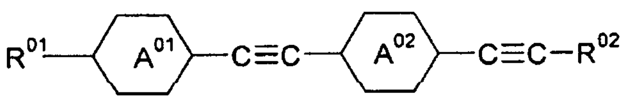 I

in which

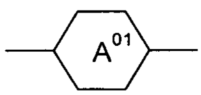

and

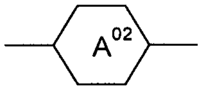,

independently of one another, denote

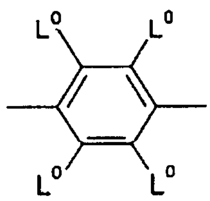, 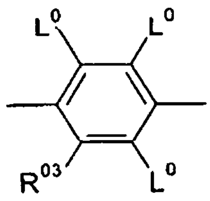, 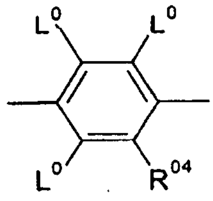,

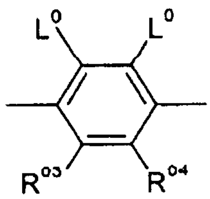, 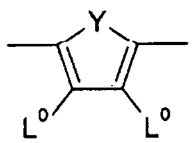, 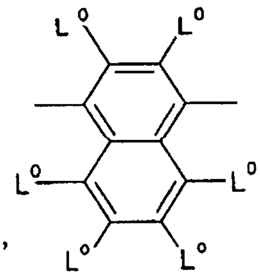,

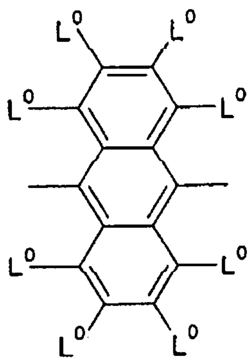, 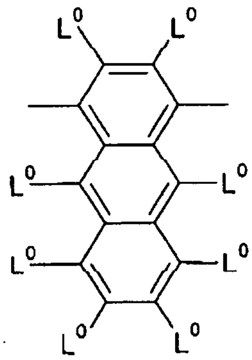, 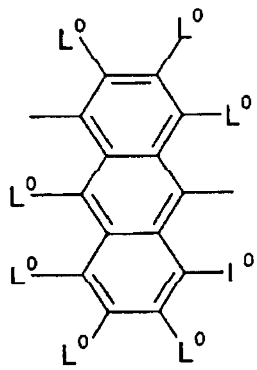

or

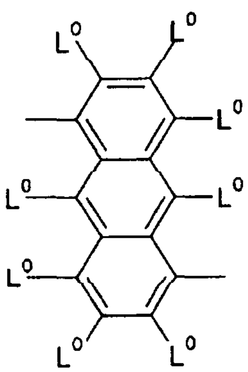,

in which Y denotes S or O,

where only one of

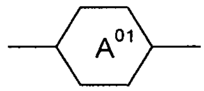

and

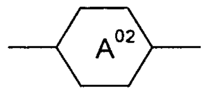

may denote

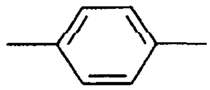

,

and in the 1,4-phenylene groups, one C-H group or two C-H groups may be replaced by N and

$L^0$ on each occurrence, independently of one another, denotes H, Br, Cl, F, -CN, -NCS, -SCN, $SF_5$, $C_1$-$C_{10}$ alkyl, $C_1$-$C_{10}$ alkoxy, $C_3$-$C_6$ cycloalkyl or a mono- or polyfluorinated $C_1$-$C_{10}$ alkyl or alkoxy group,
$R^{01}$ and $R^{02}$ each, independently of one another, denote a halogenated or unsubstituted alkyl radical having 1 to 15 C atoms, where, in addition, one or more $CH_2$ groups in these radicals may each be replaced, independently of one another, by -C≡C-, -CH=CH-, -CF=CF-, -CF=CH-, -CH=CF-, -(CO)O-, -O(CO)-, -(CO)-, -O- or -S- in such a way that O or S atoms are not linked directly to one another and, optionally, independently of one another, $R^{01}$ may also denote ethynyl (i.e. -C≡CH) and $R^{02}$ may also denote H, and
$R^{03}$ and $R^{04}$ each, independently of one another, denote a halogenated or unsubstituted alkyl radical having 1 to 6, preferably having 1 to 4, particularly preferably having 1, 2 or 3
C atoms, where, in addition, one or more $CH_2$ groups in these radicals may each be replaced, independently of one another, by -C≡C-, -CH=CH-, -CF=CF-, -CF=CH-, -CH=CF-, -(CO)O-, -O(CO)-, -(CO)-, -O- or -S- in such a way that O or S atoms are not linked directly to one another,

where, in the case where

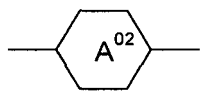

denotes

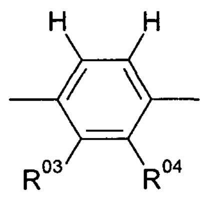

and

$R^{03}$ and $R^{04}$ both denote $CH_3$,
$R^{01}$ denotes alk-1-ynyl.

**2.** Compounds according to Claim 1, **characterised in that**

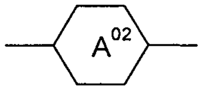

denotes

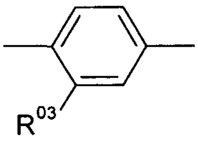 , 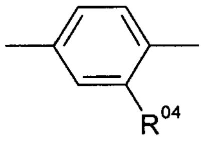 or 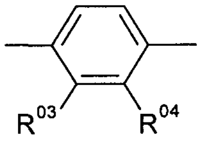 .

3. Compounds according to Claim 1 or 2, **characterised in that**

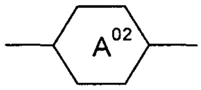

denotes

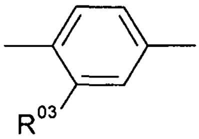 or 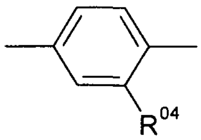 .

4. Compounds according to one or more of Claims 1 to 3 selected from the group of the compounds of the following formulae:

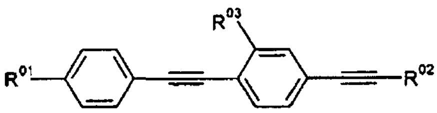

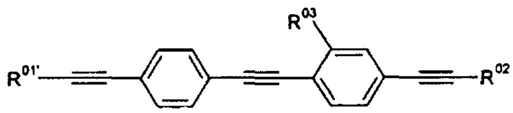

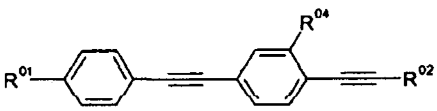

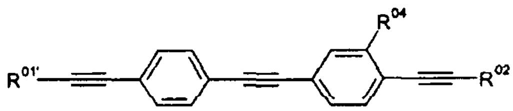

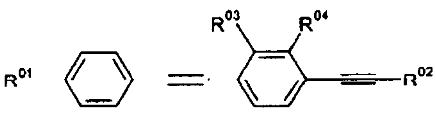

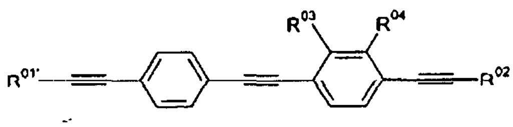

In which $R^{01}$ to $R^{04}$ have the meanings given in Claim 1.

5. Compounds according to one or more of Claims 1 to 4, **characterised in that** $R^{01}$ denotes alk-1-ynyl.

6. Liquid-crystal medium, **characterised in that** it comprises one or more compounds of the formula I according to one of Claims 1 to 5.

7. Liquid-crystal medium according to Claim 6, **characterised in that** it additionally comprises one or more compounds selected from the compounds of the formula II:

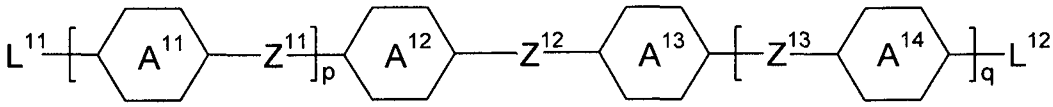 II

in which

$L^{11}$ denotes $R^{11}$ or $X^{11}$,
$L^{12}$ denotes $R^{12}$ or $X^{12}$,
$R^{11}$ and $R^{12}$, independently of one another, denote unfluorinated alkyl or unfluorinated alkoxy having 1 to 17 C atoms or unfluorinated alkenyl, unfluorinated alkynyl, unfluorinated alkenyloxy or unfluorinated alkoxyalkyl having 2 to 15 C atoms,
$X^{11}$ and $X^{12}$, independently of one another, denote F, Cl, Br, -CN, -NCS, -SCN, $-SF_5$, fluorinated alkyl or fluorinated alkoxy having 1 to 7 C atoms or fluorinated alkenyl, fluorinated alkenyloxy or fluorinated alkoxyalkyl having 2 to 7 C atoms,
p, q independently denote 0 or 1,
$Z^{11}$ to $Z^{13}$, independently of one another, denote *trans* -CH=CH-, *trans* -CF=CF-, -C=C- or a single bond, and

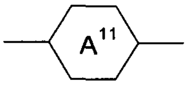 to 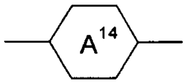 ,

independently of one another, denote

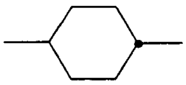 , 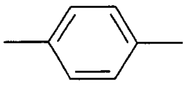 , 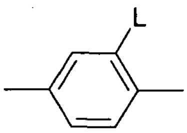 ,

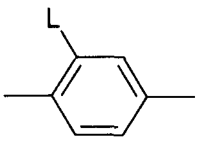 , 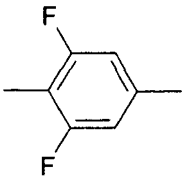 , 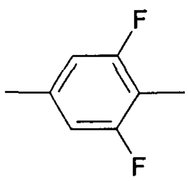 ,

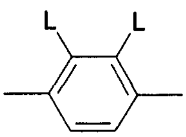 , 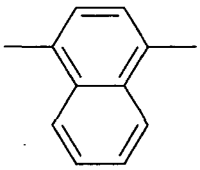 ,

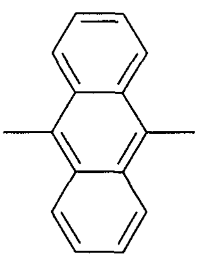 or 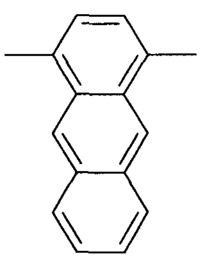 ,

and
L on each occurrence, independently of one another, denotes branched or unbranched alkyl, alkenyl or alkynyl having 1 to 12 C atoms, in which, independently of one another, one or more "$-CH_2-$" groups may also be

replaced by O, or denotes $C_3$-$C_6$ cycloalkyl, $C_3$-$C_6$ cycloalkenyl, fluorinated alkyl or alkenyl, fluorinated alkoxy or alkenyloxy, F, Cl, Br, CN, NCS, SCN or $SF_5$.

**8.** Liquid-crystal medium according to Claim 6 or 7, **characterised in that** the concentration of the compounds of the formula I in the medium is in the range from in total 5% to 95%.

**9.** Use of the compounds of the formula I according to one of Claims 1 to 5 in a liquid-crystalline medium.

**10.** Use of the compounds of the formula I according to one of Claims 1 to 5 or a liquid-crystalline medium according to one of Claims 6 to 8 in a component for high-frequency technology.

**11.** Process for the preparation of a liquid-crystal medium according to one or more of Claims 6 to 8, **characterised in that** one or more compounds of the formula I are mixed with one or more further compounds and optionally with one or more additives.

**12.** Component for high-frequency technology, **characterised in that** it comprises a liquid-crystal medium according to one or more of Claims 6 to 8.

**13.** Component according to Claim 12, **characterised in that** it is a phase shifter or a plurality of functionally connected phase shifters.

**14.** 'Phased-array' antenna, **characterised in that** it contains one or more components according to Claim 12 or 13.

**15.** Use of a liquid-crystal medium according to one or more of Claims 6 to 8 in a component for high-frequency technology.

## Revendications

**1.** Composés de la formule I :

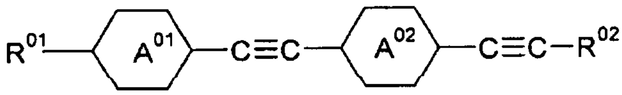

I

dans laquelle :

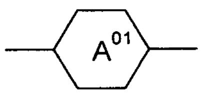

et

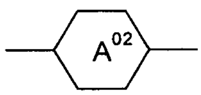

représentent, indépendamment l'un de l'autre,

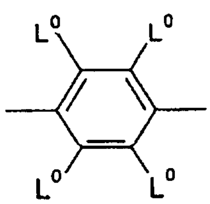

,

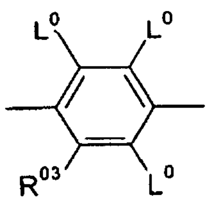

,

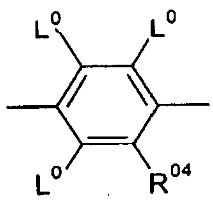

,

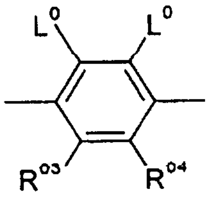,
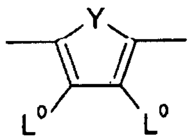,
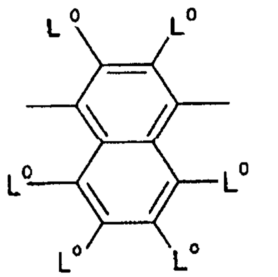,

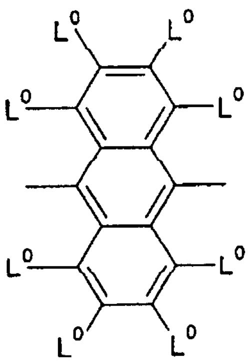,
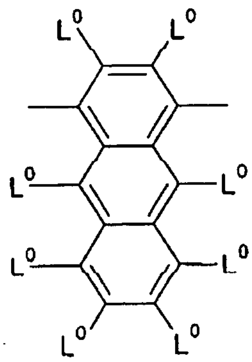,
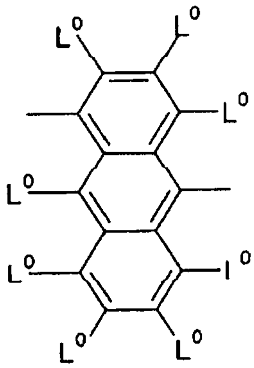

ou 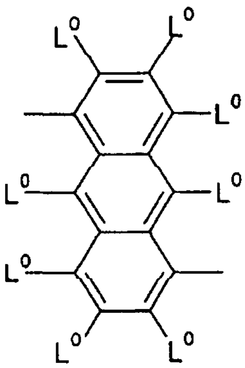,

où Y représente S ou O, où seulement l'un de

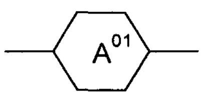

et

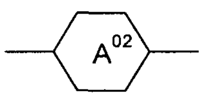

peut représenter

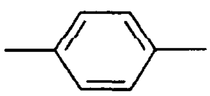,

et

dans les groupes 1,4-phénylène, un groupe C-H ou deux groupes C-H peut/peuvent être remplacé(s) par N et

$L^0$ représente, pour chaque occurrence, indépendamment les uns des autres, H, Br, Cl, F, -CN, -NCS, -SCN, $SF_5$, $C_1$-$C_{10}$ alkyle, $C_1$-$C_{10}$ alcoxy, $C_3$-$C_6$ cycloalkyle ou un groupe $C_1$-$C_{10}$ alkyle ou alcoxy monofluoré ou polyfluoré,

$R^{01}$ et $R^{02}$ représentent chacun, indépendamment l'un de l'autre, un radical alkyle halogéné ou non substitué qui comporte 1 à 15 atome(s) de C, où, en outre, un ou plusieurs groupe(s) $CH_2$ dans ces radicaux peut/peuvent chacun être remplacé(s), indépendamment les uns des autres, par -C≡C-, -CH=CH-, -CF=CF-, -CF=CH-, -CH=CF-, -(CO)O-, -O(CO)-, -(CO)-, -O-ou -S- de telle sorte que des atomes de O ou de S ne soient pas liés directement les uns aux autres et, en option, de manière indépendante, $R^{01}$ peut également représenter éthynyle (i.e. -C≡CH) et $R^{02}$ peut également représenter H, et

$R^{03}$ et $R^{04}$ représentent chacun, indépendamment l'un de l'autre, un radical alkyle halogéné ou non substitué qui comporte 1 à 6, de façon préférable qui comporte 1 à 4, de façon particulièrement préférable qui comporte 1, 2 ou 3 atome(s) de C, où, en outre, un ou plusieurs groupe(s) $CH_2$ dans ces radicaux peut/peuvent chacun être remplacé(s), indépendamment les uns des autres, par -C≡C-, -CH=CH-, -CF=CF-, -CF=CH-, -CH=CF-, -(CO)O-, -O(CO)-, -(CO)-, -O- ou -S- de telle sorte que des atomes de O ou de S ne soient pas liés directement les uns aux autres,

où, dans le cas dans lequel :

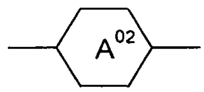

représente

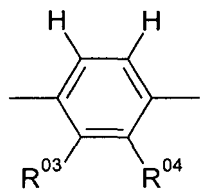

et

$R^{03}$ et $R^{04}$ représentent tous deux $CH_3$,
$R^{01}$ représente alk-1-ynyle.

2. Composés selon la revendication 1, **caractérisés en ce que**

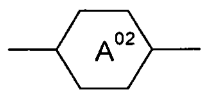

représente

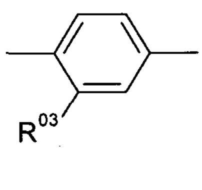

,

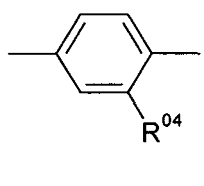

ou

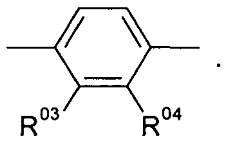

3. Composés selon la revendication 1 ou 2, **caractérisés en ce que**

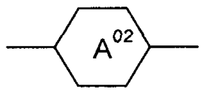

représente

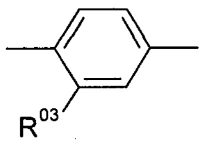

ou

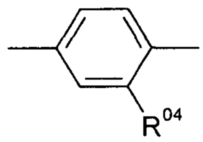

.

4. Composés selon une ou plusieurs des revendications 1 à 3 sélectionnés parmi le groupe des composés des formules qui suivent

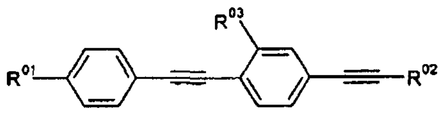

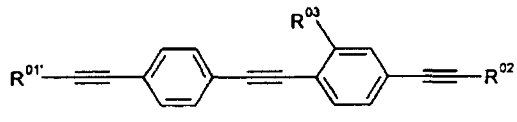

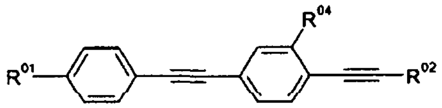

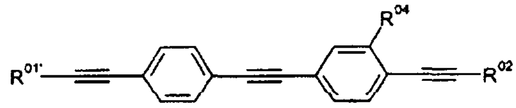

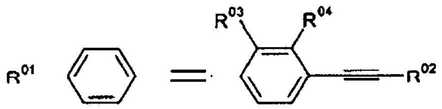

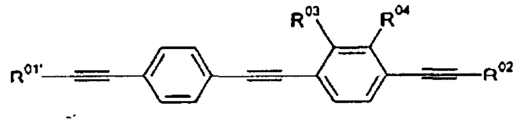

dans lesquelles $R^{01}$ à $R^{04}$ présentent les significations données selon la revendication 1.

5. Composés selon une ou plusieurs des revendications 1 à 4, **caractérisés en ce que** $R^{01}$ représente alk-1-ynyle.

6. Milieu cristallin liquide, **caractérisé en ce qu'**il comprend un ou plusieurs composé(s) de la formule I selon l'une des revendications 1 à 5.

7. Milieu cristallin liquide selon la revendication 6, **caractérisé en ce qu'**il comprend de manière additionnelle un ou plusieurs composé(s) sélectionné(s) parmi les composés de la formule II :

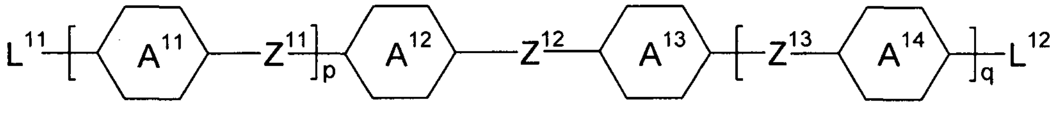

II

dans laquelle :

$L^{11}$ représente $R^{11}$ ou $X^{11}$,
$L^{12}$ représente $R^{12}$ ou $X^{12}$,
$R^{11}$ et $R^{12}$ représentent, indépendamment l'un de l'autre, alkyle non fluoré ou alcoxy non fluoré qui comporte

1 à 17 atome(s) de C ou alkényle non fluoré, alkynyle non fluoré, alkényloxy non fluoré ou alcoxyalkyle non fluoré qui comporte 2 à 15 atomes de C,
$X^{11}$ et $X^{12}$ représentent, indépendamment l'un de l'autre, F, Cl, Br, -CN, -NCS, -SCN, $-SF_5$, alkyle fluoré ou alcoxy fluoré qui comporte 1 à 7 atome(s) de C ou alkényle fluoré, alkényloxy fluoré ou alcoxyalkyle fluoré qui comporte 2 à 7 atomes de C,
p, q représentent, de manière indépendante, 0 ou 1,
$Z^{11}$ à $Z^{13}$ représentent, indépendamment les uns des autres, *trans* -CH=CH-, *trans* -CF=CF-, -C=C- ou une liaison simple, et

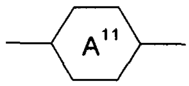
à
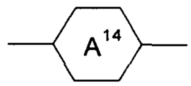

représentent, indépendamment les uns des autres,

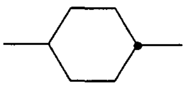
,
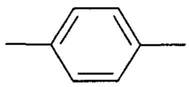
,
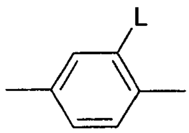
,

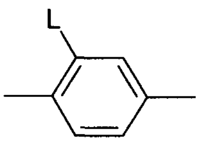
,
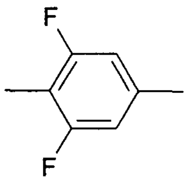
,
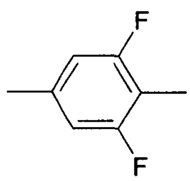
,

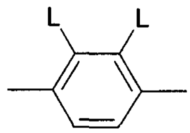
,
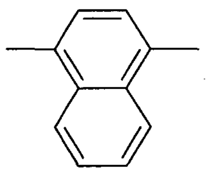
,

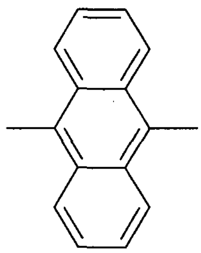
ou
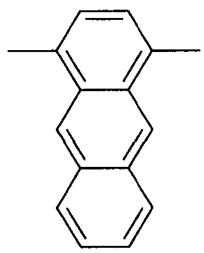
,

et
L représente, pour chaque occurrence, indépendamment les uns des autres, alkyle, alkényle ou alkynyle ramifié ou non ramifié qui comporte 1 à 12 atome(s) de C, où, indépendamment les uns des autres, un ou plusieurs groupe(s) "$-CH_2-$" peut/peuvent également être remplacé(s) par O, ou représente $C_3$-$C_6$ cycloalkyle, $C_3$-$C_6$ cycloalkényle, alkyle ou alkényle fluoré, alcoxy ou alkényloxy fluoré, F, Cl, Br, CN, NCS, SCN ou $SF_5$.

8. Milieu cristallin liquide selon la revendication 6 ou 7, **caractérisé en ce que** la concentration des composés de la formule I dans le milieu est dans la plage de, au total, 5 % à 95 %.

9. Utilisation des composés de la formule I selon l'une des revendications 1 à 5 dans un milieu cristallin liquide.

10. Utilisation des composés de la formule I selon l'une des revendications 1 à 5 ou d'un milieu cristallin liquide selon l'une des revendications 6 à 8 dans un composant pour une technologie des hautes fréquences.

11. Procédé pour la préparation d'un milieu cristallin liquide selon une ou plusieurs des revendications 6 à 8, **caractérisé en ce qu'**un ou plusieurs composé(s) de la formule I est/sont mélangé(s) avec un ou plusieurs autre(s) composé(s) et en option, avec un ou plusieurs additif(s).

12. Composant pour une technologie des hautes fréquences, **caractérisé en ce qu'**il comprend un milieu cristallin liquide selon une ou plusieurs des revendications 6 à 8.

13. Composant selon la revendication 12, **caractérisé en ce qu'**il s'agit d'un déphaseur ou d'une pluralité de déphaseurs connectés fonctionnellement.

14. Antenne 'réseau à commande de phase', **caractérisée en ce qu'**elle contient un ou plusieurs composant(s) selon la revendication 12 ou 13.

15. Utilisation d'un milieu cristallin liquide selon une ou plusieurs des revendications 6 à 8 dans un composant pour une technologie des hautes fréquences.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102004029429 A
- JP 2005120208 A
- JP 5255151 A
- WO 2009125721 A1
- JP 10045642 A
- DE 10120024
- EP 0377516 A
- DE 102011112950 A1


### In der Beschreibung aufgeführte Nicht-Patentliteratur


- **A. GAEBLER ; A. MOESSINGER ; F. GOELDEN et al.** Liquid Crystal-Reconfigurable Antenna Concepts for Space Applications at Microwave and Millimeter Waves. *International Journal of Antennas and Propagation,* 2009, vol. 2009, 7
- **A. PENIRSCHKE ; S. MÜLLER ; P. SCHEELE ; C. WEIL ; M. WITTEK ; C. HOCK ; R. JAKOBY.** Cavity Perturbation Method for Characterization of Liquid Crystals up to 35 GHz. *34th European Microwave Conference - Amsterdam,* 545-548
- **DRUCKSCHRIFTEN SHIN-TSON WU et al.** *Jpn. J. Appl. Phys.,* 1999, vol. 38, 286-288
- **SHIN-TSON WU et al.** *Jpn. J. Appl. Phys.,* 2000, vol. 39, 38-41
- Merck Liquid Crystals, Physical Properties of Liquid Crystals. Merck KGaA, November 1997
- **A. PENIRSCHKE et al.** Cavity Perturbation Method for Characterization of Liquid Crystals up to 35GHz. *34th European Microwave Conference,* 545-548
- Direct Simulation of Material Permittivities ... **A. GAEBLER et al.** 12MTC 2009 - International Instrumentation and Measurement Technology Conference. IEEE, 2009, 463-467
- **A. PENIRSCHKE et al.** *34th European Microwave Conference - Amsterdam,* 545-548